# EUROPEAN PATENT SPECIFICATION

(11) **EP 1 513 580 B1**
(45) Date of publication and mention of the grant of the patent: **18.03.2009**
(21) Application number: 03738970.7
(22) Date of filing: 27.05.2003
(51) Int. Cl.: A61M 5/145, A61M 5/315, F03G 7/06

(54) **PLUNGER ASSEMBLY FOR PATIENT INFUSION DEVICE**
KOLBENANORDNUNG FÜR EINE PATIENTENINFUSIONSVORRICHTUNG
ENSEMBLE PISTON POUR DISPOSITIF DE PERFUSION POUR MALADES

(30) Priority: 06.06.2002 US 163688
(43) Date of publication of application: 16.03.2005
(73) Proprietor: Insulet Corporation, Beverly, MA 01915-6120 (US)
(72) Inventor: FLAHERTY, Christopher, J., Topsfield, MA 01983 (US)
(74) Representative: Hill, Justin John
(86) International application number: PCT/US2003/016545
(87) International publication number: WO 2003/103763

(56) References cited:
- WO-A-02/20073
- US-A- 4 766 889
- US-A- 4 944 659
- US-A- 5 222 362
- US-A- 6 050 457
- US-A- 6 159 188
- US-A1- 2001 016 710
- US-A1- 2003 097 092

## Description

### Field of the Invention

The present invention relates generally to medical devices and systems, and more particularly to small, low cost, portable infusion devices that are useable to achieve precise, sophisticated, and programmable flow patterns for the delivery of therapeutic liquids such as insulin to a mammalian patient. Even more particularly, the present invention is directed to a spring-driven plunger assembly for a fluid delivery device, that can utilize shape memory elements.

### Background of the Invention

Today, there are numerous diseases and other physical ailments that are treated by various medicines including pharmaceuticals, nutritional formulas, biologically derived or active agents, hormonal and gene based material and other substances in both solid or liquid form. In the delivery of these medicines, it is often desirable to bypass the digestive system of a mammalian patient to avoid degradation of the active ingredients caused by the catalytic enzymes in the digestive tract and liver. Delivery of a medicine other than by way of the intestines is known as parenteral delivery. Parenteral delivery of various drugs in liquid form is often desired to enhance the effect of the substance being delivered, insuring that the unaltered medicine reaches its intended site at a significant concentration. Also, undesired side effects associated with other routes of delivery, such as systemic toxicity, can potentially be avoided.

Often, a medicine may only be available in a liquid form, or the liquid version may have desirable characteristics that cannot be achieved with solid or pill form. Delivery of liquid medicines may best be accomplished by infusing directly into the cardiovascular system via veins or arteries, into the subcutaneous tissue or directly into organs, tumors, cavities, bones or other site specific locations within the body.

Parenteral delivery of liquid medicines into the body is often accomplished by administering bolus injections using a needle and reservoir, or continuously by gravity driven dispensers or transdermal patch technologies. Bolus injections often imperfectly match the clinical needs of the patient, and usually require larger individual doses than are desired at the specific time they are given. Continuous delivery of medicine through gravity feed systems compromise the patient's mobility and lifestyle, and limit the therapy to simplistic flow rates and profiles. Transdermal patches have special requirements of the medicine being delivered, particularly as it relates to the molecular structure, and similar to gravity feed systems, the control of the drug administration is severely limited.

Ambulatory infusion pumps have been developed for delivering liquid medicaments to a patient. These infusion devices have the ability to offer sophisticated fluid delivery profiles accomplishing bolus requirements, continuous infusion and variable flow rate delivery. These infusion capabilities usually result in better efficacy of the drug and therapy and less toxicity to the patient's system. An example of a use of an ambulatory infusion pump is for the delivery of insulin for the treatment of diabetes mellitus. These pumps can deliver insulin on a continuous basal basis as well as a bolus basis as is disclosed in U.S. Patent 4,498,843 to Schneider et al.

The ambulatory pumps often work with a reservoir to contain the liquid medicine, such as a cartridge, a syringe or an IV bag, and use electro-mechanical pumping or metering technology to deliver the medication to the patient via tubing from the infusion device to a needle that is inserted transcutaneously, or through the skin of the patient. The devices allow control and programming via electromechanical buttons or switches located on the housing of the device, and accessed by the patient or clinician. The devices include visual feedback via text or graphic screens, such as liquid crystal displays known as LCD's, and may include alert or warning lights and audio or vibration signals and alarms. The device can be worn in a harness or pocket or strapped to the body of the patient.

Currently available ambulatory infusion devices are expensive, difficult to program and prepare for infusion, and tend to be bulky, heavy and very fragile. Filling these devices can be difficult and require the patient to carry both the intended medication as well as filling accessories. The devices require specialized care, maintenance, and cleaning to assure proper functionality and safety for their intended long term use. Due to the high cost of existing devices, healthcare providers limit the patient populations approved to use the devices and therapies for which the devices can be used.

Clearly, therefore, there was a need for a programmable and adjustable infusion system that is precise and reliable and can offer clinicians and patients a small, low cost, light-weight, easy-to-use alternative for parenteral delivery of liquid medicines.

In response, the applicant of the present application provided a small, low cost, light-weight, easy-to-use device for delivering liquid medicines to a patient. The device, which is described in detail in co-pending U. S. application serial No. 09/943,992, filed on August 31,2001, includes an exit port, a dispenser for causing fluid from a reservoir to flow to the exit port, a local processor programmed to cause a flow of fluid to the exit port based on flow instructions from a separate, remote control device, and a wireless receiver connected to the local processor for receiving the flow instructions. To reduce the size, complexity and costs of the device, the device is provided with a housing that is free of user input components, such as a keypad, for providing flow instructions to the local processor.

What are still desired are new and improved components, such as plunger assemblies and reservoirs, for a device for delivering fluid to a patient. Preferably, the components will be simple in design, and relatively compact, lightweight, easy to manufacture and inexpensive, such that the resulting fluid delivery device can be effective, yet inexpensive and disposable.
US-A-5 222 362 discloses a heat-activated fluid delivery system using thermal actuators in which energy is derived from the physical expansion of paraffin wax as it changes state from solid to liquid when heated.

### Summary of the Invention

The present invention provides a device for delivering fluid, such as insulin for example, to a patient. The subject-matter of the invention in defined by claim 1. In addition, further advantageous embodiment follow from the dependent claims.

The invention together with additional features and advantages thereof may best be understood by reference to the following detailed descriptions and examples taken in connection with the accompanying illustrated drawings.

### Brief Description of the Drawings

Fig. 1 is a perspective view of a first exemplary embodiment of a fluid delivery device constructed in accordance with the present invention and shown secured on a patient, and a remote control device for use with the fluid delivery device (the remote control device being enlarged with respect to the patient and the fluid delivery device for purposes of illustration);

Fig. 2 is a sectional side view of the fluid delivery device of Fig. 1 showing an exemplary embodiment of a plunger assembly constructed in accordance with the present invention for causing fluid to be dispensed from the device;

Figs. 3a-3f are enlarged sectional side views illustrating operation of the plunger assembly of Fig. 2;

Fig. 4 is a sectional side view of another exemplary embodiment of a reservoir and a plunger assembly constructed in accordance with the present invention for use with the fluid delivery device of Fig. 1;

Fig. 5 is a top plan view of a longitudinal reference guide attached to the reservoir of Fig. 4;

Fig. 6 is an enlarged sectional side view of the plunger assembly of Fig. 4;

Figs. 7a-7e are enlarged sectional side views illustrating operation of another exemplary embodiment of a plunger assembly constructed in accordance with the present invention for use with the fluid delivery device of Fig. 1;

Figs. 8a-8c are enlarged sectional side views illustrating operation of an exemplary plunger assembly not forming part of present invention for use with the fluid delivery device of Fig. 1; and

Fig. 9 is a sectional side view of a fluid delivery device similar to the fluid delivery device of Fig. 2 showing another exemplary embodiment of a reservoir and a plunger assembly constructed in accordance with the present invention for causing fluid to be dispensed from the device.

Like reference characters designate identical or corresponding components and units throughout the several views.

### Detailed Description of Exemplary Embodiments

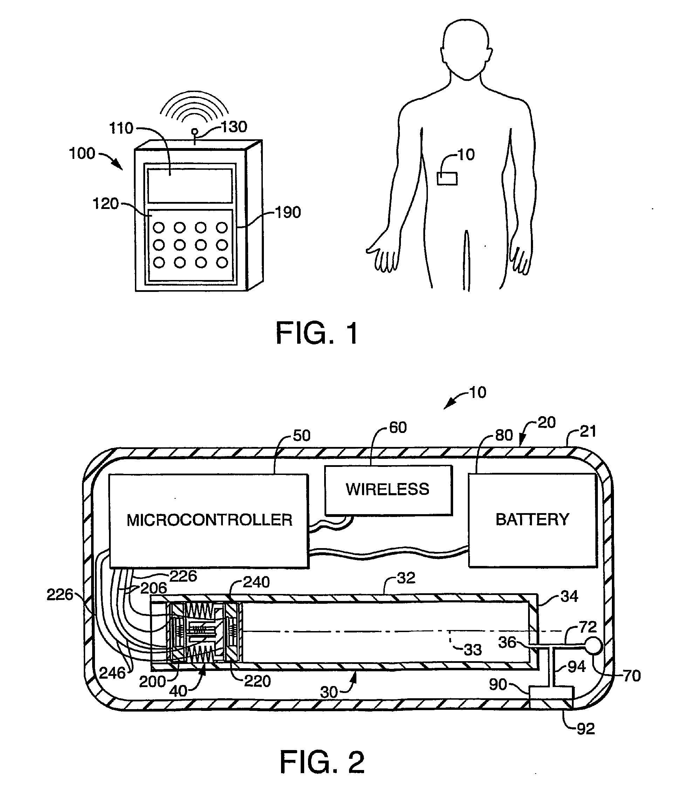

Referring first to Fig. 2, there is illustrated an exemplary embodiment of a fluid delivery device 10 including a dispenser in the form of a plunger assembly 40 constructed in accordance with the present invention. The plunger assembly 40 causes fluid flow from a reservoir 30 to an exit port assembly 70 during operation of the device 10. In general, the plunger assembly 40 is spring-driven and can use shape memory elements in accordance with the present invention to provide effective, yet simple and inexpensive fluid dispensing for fluid delivery devices.

The fluid delivery device 10 of Fig. 2 can be used for the delivery of fluids to a person or animal. The types of liquids that can be delivered by the fluid delivery device 10 include, but are not limited to, insulin, antibiotics, nutritional fluids, total parenteral nutrition or TPN, analgesics, morphine, hormones or hormonal drugs, gene therapy drugs, anticoagulants, analgesics, cardiovascular medications, AZT or chemotherapeutics. The types of medical conditions that the fluid delivery device 10 might be used to treat include, but are not limited to, diabetes, cardiovascular disease, pain, chronic pain, cancer, AIDS, neurological diseases, Alzheimer's Disease, ALS, Hepatitis, Parkinson's Disease or spasticity. In addition, it should be understood that the plunger assembly 40 according to the present invention can be used with fluid delivery devices other than those used for the delivery of fluids to persons or animals.

The fluid delivery device 10 also includes a processor or electronic microcontroller (hereinafter referred to as the "local" processor) 50 connected to the plunger assembly 40. The local processor 50 is programmed to cause a flow of fluid to the exit port assembly 70 based on flow instructions from a separate, remote control device 100, an example of which is shown in Fig. 1. Referring also to Fig. 2, the fluid delivery device 10 further includes a wireless receiver 60 connected to the local processor 50 for receiving the flow instructions from the separate, remote control device 100 and delivering the flow instructions to the local processor. The device 10 also includes a housing 20 containing the exit port assembly 70, the reservoir 30, the plunger assembly 40, the local processor 50 and the wireless receiver 60.

As shown, the housing 20 of the fluid delivery device 10 is free of user input components for providing flow instructions to the local processor 50, such as electromechanical switches or buttons on an outer surface 21 of the housing, or interfaces otherwise accessible to a user to adjust the programmed flow rate through the local processor 50. The lack of user input components allows the size, complexity and costs of the device 10 to be substantially reduced so that the device 10 lends itself to being small and disposable in nature. Examples of such devices are disclosed in co-pending U.S. patent application serial number 09/943,992, filed on August 31, 2001 (Atty. Docket No. INSL-110), and entitled DEVICES, SYSTEMS AND METHODS FOR PATIENT INFUSION, which is assigned to the assignee of the present application.

In order to program, adjust the programming of, or otherwise communicate user inputs to the local processor 50, the fluid delivery device 10 includes the wireless communication element, or receiver 60 for receiving the user inputs from the separate, remote control device 100 of Fig. 1. Signals can be sent via a communication element (not shown) of the remote control device 100, which can include or be connected to an antenna 130, shown in Fig. 1 as being external to the device 100.

The remote control device 100 has user input components, including an array of electromechanical switches, such as the membrane keypad 120 shown. The control device 100 also includes user output components, including a visual display, such as a liquid crystal display (LCD) 110. Alternatively, the control device can be provided with a touch screen for both user input and output. Although not shown in Fig. 1, the remote control device 100 has its own processor (hereinafter referred to as the "remote" processor) connected to the membrane keypad 120 and the LCD 110. The remote processor receives the user inputs from the membrane keypad 120 and provides "flow" instructions for transmission to the fluid delivery device 10, and provides information to the LCD 110. Since the remote control device 100 also includes a visual display 110, the fluid delivery device 10 can be void of an information screen, further reducing the size, complexity and costs of the device 10.

The communication element 60 of the device 10 preferably receives electronic communication from the remote control device 100 using radio frequency or other wireless communication standards and protocols. In a preferred embodiment, the communication element 60 is a two-way communication element, including a receiver and a transmitter, for allowing the fluid delivery device 10 to send information back to the remote control device 100. In such an embodiment, the remote control device 100 also includes an integral communication element comprising a receiver and a transmitter, for allowing the remote control device 100 to receive the information sent by the fluid delivery device 10.

The local processor 50 of the device 10 contains all the computer programs and electronic circuitry needed to allow a user to program the desired flow patterns and adjust the program as necessary. Such circuitry can include one or more microprocessors, digital and analog integrated circuits, resistors, capacitors, transistors and other semiconductors and other electronic components known to those skilled in the art. The local processor 50 also includes programming, electronic circuitry and memory to properly activate the plunger assembly 40 at the needed time intervals.

In the exemplary embodiment of Fig. 2, the device 10 includes a power supply 80, such as a battery or capacitor, for supplying power to the local processor 50. The power supply 80 is preferably integrated into the fluid delivery device 10, but can be provided as replaceable, e.g., a replaceable battery.

Although not shown, the device 10 can include sensors or transducers such as a reservoir volume transducer or a reservoir pressure transducer, for transmitting information to the local processor 50 to indicate how and when to activate the plunger assembly 40, or to indicate other parameters determining flow, pump flow path prime condition, blockage in flow path, contact sensors, rotary motion or other motion indicators, as well as conditions such as the reservoir 30 being empty or leaking, or the dispensing of too much or too little fluid from the reservoir, etc.

The volume of the reservoir 30 is chosen to best suit the therapeutic application of the fluid delivery device 10 impacted by such factors as available concentrations of medicinal fluids to be delivered, acceptable times between refills or disposal of the fluid delivery device 10, size constraints and other factors. The reservoir 30 may be prefilled by the device manufacturer or a cooperating drug manufacturer, or may include external filling means, such as a fill port 90 having a needle insertion septum 92 as shown in Fig. 2, for example. In addition, or alternatively, the device 10 can be provided with a removable and replaceable reservoir.

Although not shown, the exit port assembly 70 can include elements to penetrate the skin of the patient, such that the entire volume of the flow path of the fluid delivery device 10 is predetermined. For example, a needle-connection tubing terminating in a skin penetrating cannula (not shown) can be provided as an integral part of the exit port assembly 70, with the skin penetrating cannula comprising a rigid member, such as a needle. The exit port assembly 70 can further be provided with injection means, such as a spring driven mechanism, to assist in penetrating the skin with the skin penetrating cannula. For example, if the cannula is a flexible tube, a rigid penetrator within the lumen of the tube can be driven through the skin by the injection means and then withdrawn, leaving the soft cannula in place in the subcutaneous tissue of the patient or other internal site. The injection means may be integral to the device 10, or removable soon after transcutaneous penetration.

Alternatively, the exit port assembly 70 can be adapted to connect, with a Luer connector for example, to a separate, standard infusion device that includes a skin penetrating cannula. In any event, the exit port assembly 70 can also be provided with a removable plug (not shown) for preventing leakage during storage and shipment if prefilled, and during priming if filled by user, and prior to use. It should be understood that, as used herein, the term "flow path" is meant to include all portions of the fluid delivery device 10 that contain therapeutic fluid for delivery to a patient, e.g., all portions between the fill port of the reservoir to the tip of the needle of the exit port assembly.

Although not shown, the device 10 can also be provided with an adhesive layer on the outer surface of the housing 20 for securing the device 10 directly to the skin of a patient. The adhesive layer is preferably provided in a continuous ring encircling the exit port assembly 70 in order to provide a protective seal around the penetrated skin. The housing 20 can be made from flexible material, or can be provided with flexible hinged sections that allow the fluid delivery device 10 to flex during patient movement to prevent detachment and aid in patient comfort.

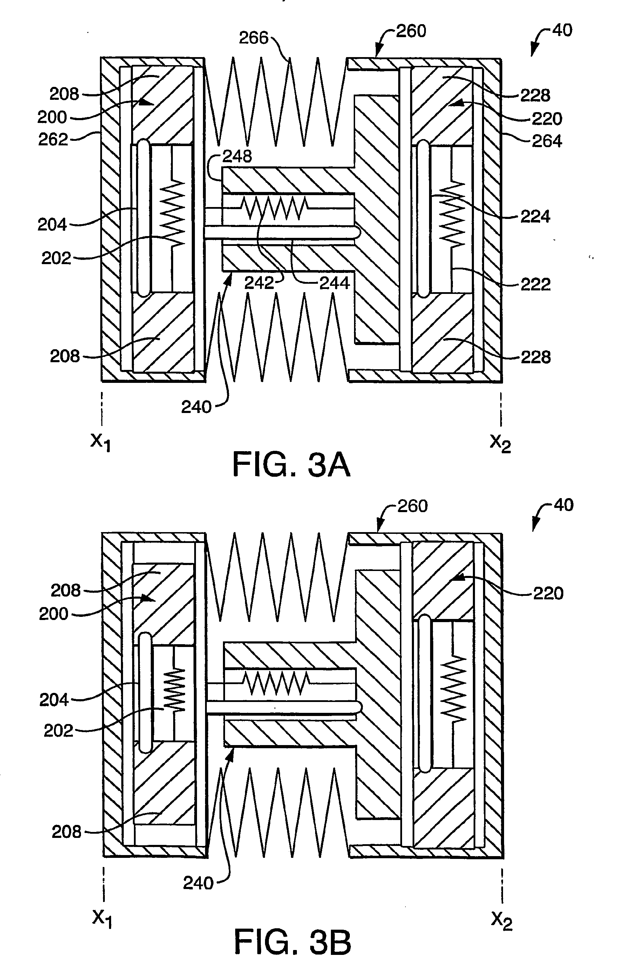

Referring to Figs. 2 and 3a-3f, the present disclosure provides the plunger assembly 40 and the reservoir 30 for use with the fluid delivery device 10 of Figs. 1 and 2. The plunger assembly 40 is small and simple in design, and inexpensive and easy to manufacture, in order to further reduce the size, complexity and costs of the fluid delivery device 10, such that the device 10 continues to lend itself to being small and disposable in nature. In general, the device 10 is provided with a non-pressurized, syringe-like reservoir 30, and the plunger assembly 40 operates to cause flow from the reservoir 40 to the exit port assembly 70. The plunger assembly 40 is controlled by the local processor 50, which includes electronic programming, controls, and circuitry to allow sophisticated fluid delivery programming and control of the plunger assembly 40.

Referring to Fig. 2, the syringe-like reservoir 30 is provided with a side wall 32 extending along a longitudinal axis 33 between an open end and an end wall 34 of the reservoir. The end wall 34 includes an outlet, or an opening 36 that functions as an outlet and an inlet, connected through a first lumen 72 to the exit port assembly 70 and connected through a second lumen 94 to the fill port 90. The plunger assembly 40 is received in the reservoir 30 and is shaped and sized such that a fluid-tight seal is generally formed between at least a portion of the plunger assembly 40 and the side wall 32 of the reservoir so that movement of the plunger assembly 40 towards the end wall 34 of the reservoir 30 forces fluid through the outlet 36 to the exit port assembly 70.

The plunger assembly 40 is prevented from rotating with respect to the side wall 32. For example, the reservoir 30 and the plunger assembly 40 are provided with matching non-circular cross-sections, such as oval cross-sections. Alternatively, the plunger assembly 40 can be provided with at least one longitudinal channel and the side wall 32 of the reservoir 30 can be provided with at least one protrusion extending longitudinally along its length and received within the channel of the plunger assembly (or vice versa) to prevent rotation of the plunger assembly. In addition, the reservoir 30 and the plunger assembly 40 can alternatively be provided with other matching non-circular cross-sections, such as oval, square or rectangular, along at least a portion of their length to prevent rotation of the plunger assembly 40 with respect to the side wall 32, without the use of a protrusion and a channel. Such non-circular cross-sections can also include simply providing the side wall 32 and the plunger assembly 40 with mating flat portions in otherwise circular cross-sections. The side wall 32 and the end wall 34 of the reservoir are preferably made from a rigid material such as a suitable metal (e.g., stainless steel) or plastic. The plunger assembly 40, however, does not need to be prevented from rotating with respect to the side wall 32.

The plunger assembly 40 includes a first lateral segment 200 extending laterally with respect to the longitudinal axis 33 of the reservoir 30 and contacting the side wall 32 of the reservoir, and a second lateral segment 220 extending laterally with respect to the longitudinal axis 33 of the reservoir 30 and contacting the side wall 32 of the reservoir. The second lateral segment 220 is positioned between the first lateral segment 200 and the outlet 36 of the reservoir 30 and is longitudinally spaced from the first lateral segment 200. The plunger assembly 40 also includes a longitudinal segment 240 extending substantially parallel with respect to the longitudinal axis 33 of the reservoir and connecting the first and the second lateral segments 200, 220.

The longitudinal segment 240 includes a spring 242 biasing the first and the second lateral segments 200, 220 longitudinally apart, and an actuator 244 arranged to overcome the spring and bias the first and the second lateral segments 200, 220 longitudinally together upon actuation. In the exemplary embodiment shown, the spring comprises a helical (or coil) compression spring 242 that is made of a suitable material such as stainless steel or a plastic. The spring 242, however, can be provided in other forms for biasing the first and the second lateral segments 200, 220 longitudinally apart, such as buckling columns, spring washers, or Bellville spring washers for example.

In the exemplary embodiment of the plunger assembly 40 of the present invention as shown in Figs. 2 and 3a-3f the actuator is provided as a shape memory element 244 made of a shape memory material. Alternatively, the actuator can be provided in the form of a solenoid, a piezoelectric element, or another actuator capable of bringing the first and the second lateral segments 200, 220 together against the force of the spring 242 upon being actuated.

The application of an electrical current to the shape memory element 244 heats the material and results in molecular and crystalline restructuring of the shape memory material. If the shape memory material is in the shape of an elongated wire, for example, as the shape memory element 244 preferably is, this restructuring causes a decrease in length. Nitinol, a well-known alloy of nickel and titanium, is an example of such a so-called shape memory material and is preferred for use as the shape memory element 244.

In general, when the shape memory element 244 is in its martensitic form (i.e., low temperature state), it is easily deformed to an elongated shape by the spring. However, when the alloy is heated through its transformation temperatures, the shape memory element 244 reverts to its austenite form (ie., high temperature state) and recovers its shorter, original shape with great force. The temperature (or the level of electrical charge) at which the alloy remembers its high temperature form can be adjusted by slight changes in alloy composition and through heat treatment. In the nickel-titanium alloys, for instance, austenite temperature can be changed from above 100° C to below 100° C. The shape recovery process occurs over a range of just a few degrees and the start or finish of the transformation can be controlled to within a degree or two if necessary.

These unique alloys also show a superelastic behavior if deformed at a temperature which is slightly above their transformation temperatures. This effect is caused by the stress-induced formation of some martensite above its normal temperature. Because it has been formed above its normal temperature, the martensite reverts immediately to undeformed austenite as soon as the stress is removed. This process provides a very springy, "rubberlike" elasticity in these alloys. A one-way SME alloy can be deformed, then recover to retain permanently its original shape when heated to a certain temperature. A two-way alloy, however, holds its original shape at one temperature and takes on another shape at a different temperature. Two-way memory is unique in that the material "remembers" different high temperature and low temperature shapes.

The shape memory element 244 of the embodiment of the present invention comprises a one-way shape memory alloy. However, it should be understood that the shape memory element 244 of the longitudinal segment 240 can be provided as a two-way shape memory alloy if desired. As shown Figs. 2 and 3a-3f, the shape memory element 244 is secured between the first and the second lateral segments 200, 220 of the plunger assembly 40. As shown in Fig. 2, the fluid delivery device 10 includes wires 246 connecting opposite ends of the shape memory element 244 to the processor 50, such that the processor can apply electrical charges to the shape memory element 244 for controlling the longitudinal segment 240.

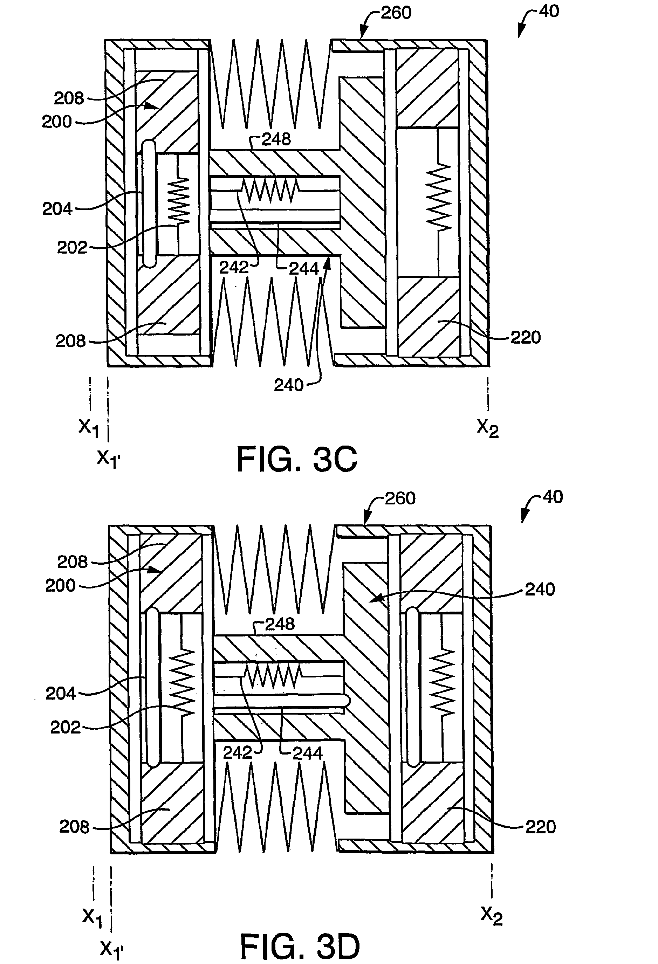

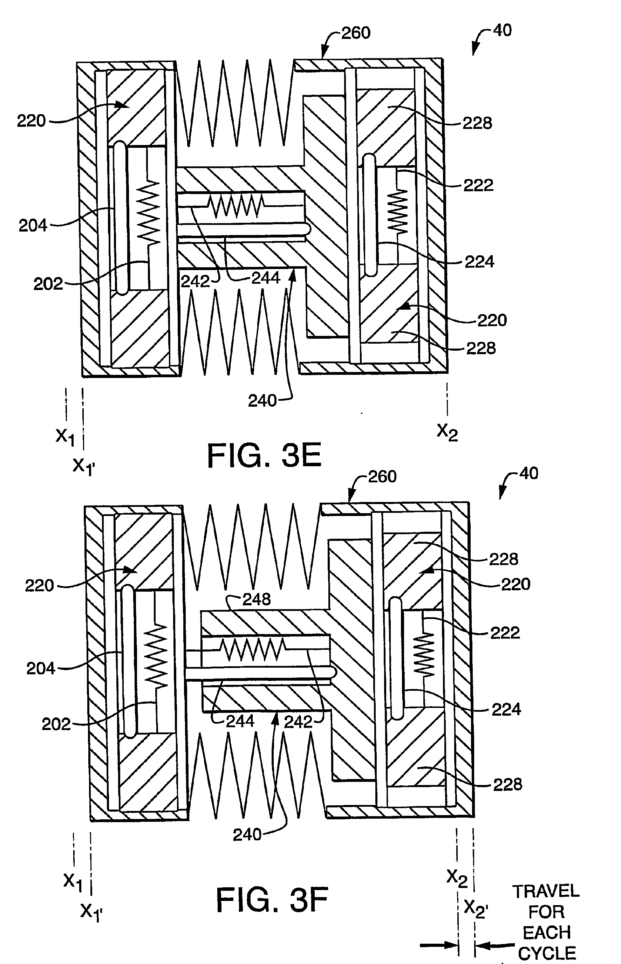

When a charge is applied to the elongated shape memory element 244 through the wires 246, the length of the shape memory element 244 decreases from an uncharged length to a charged length. The shape memory element 244 is arranged such that the changeable length of the shape memory element 244 decreasing from an uncharged length to a charged length causes the first and the second lateral segments 200, 220 to be drawn together against the force of the spring 242, as shown in Figs. 3c, 3d and 3e. When the charge is removed from the elongated shape memory element 244, the spring 242 is allowed to bias the first and the second lateral segments 200, 220 apart and increase the length of the shape memory element 244 from the charged length to the uncharged length, as shown in Figs. 3f, 3a and 3b.

The longitudinal segment 240 of the plunger assembly 40 also includes a rigid, longitudinally extending projection 248 that limits the smallest longitudinal distance that can be attained between the first and the second lateral segments 200, 220 upon actuation of the shape memory element 244 (i.e., when the first and the second lateral segments 200, 220 are pulled together by the charged shape memory element 244). The differences in lengths between the fully elongated and uncharged shape memory element 244 and the longitudinally extending projection 248 defines the distance traveled by the plunger assembly 40 during a cycle of charges, as described in greater detail below.

In the embodiment of Figs. 2 and 3a-3f, the first lateral segment 200 includes two blocks 208 laterally movable with respect to the longitudinal axis 33 of the reservoir 30, and a spring 202 biasing the two blocks 208 apart and laterally outwardly to frictionally engage the side wall 32 of the reservoir 30 and prevent longitudinal movement of the first lateral segment 200 within the reservoir 30. In the exemplary embodiment shown, the spring comprises a helical compression spring 202 but can be provided in other suitable forms for biasing the blocks 208 laterally apart. The blocks can be of various shapes including cylindrical pins, rectangular pins, wedge portions, partial rings, etc., and can be made from various rigid or semi-rigid materials. In any event, the frictional forces created between the blocks 208 and the side wall 32 must be greater than the force generated by the actuator 244 of the longitudinal segment 240.

The first lateral segment 200 also includes an actuator 204 arranged to overcome the spring 202 and bias the blocks 208 together laterally upon actuation. When biased together by the actuator 204, the blocks 208 disengage from the side wall 32 of the reservoir 30 to allow longitudinal movement of the first lateral segment 200 within the reservoir 30. The actuator of the first lateral segment 200 is provided as a shape memory element 204 made of a shape memory material. Alternatively, the actuator can be provided in the form of a solenoid, a piezoelectric element, or another actuator capable of bringing the blocks 208 together against the force of the spring 202 upon being actuated. The shape memory element 204 of the first lateral segment 200 comprises a one-way shape memory alloy. However, the shape memory element 204 can be provided as a two-way shape memory alloy. As shown Figs. 2 and 3a-3f, the elongated shape memory element 204 is secured at opposing ends between the two blocks 208 and is anchored to the first lateral segment 200 at a midpoint between the two blocks 208. As shown in Fig. 2, the fluid delivery device 10 includes wires 206 connecting the opposite ends of the shape memory element 204 to the processor 50 such that the processor can apply an electrical charge to the element 204.

When a charge is applied to the elongated shape memory element 204 of the first lateral segment 200, the length of the shape memory element 204 decreases from an uncharged length to a charged length. The shape memory element 204 of the first lateral segment 200 is arranged such that the changeable length of the shape memory element 204 decreasing from an uncharged length to a charged length causes the two blocks 208 to be drawn together against the force of the spring 202, as shown in Figs. 3b and 3c. When the charge is removed from the elongated shape memory element 204, the spring 202 is allowed to bias the two blocks 208 apart and increase the length of the shape memory element 204 from the charged length to the uncharged length, as shown in Figs. 3a and 3d-3f.

In the embodiment of Figs. 2 and 3a-3f, the second lateral segment 220 also includes two blocks 228 laterally movable with respect to the longitudinal axis 33 of the reservoir 30, and a spring 222 biasing the two blocks 228 apart and laterally outwardly to frictionally engage the side wall 32 of the reservoir 30 and prevent longitudinal movement of the second lateral segment 220 within the reservoir 30. In the exemplary embodiment shown, the spring comprises a helical compression spring 222 but can be provided in other suitable forms for biasing the blocks 228 laterally apart.

The second lateral segment 220 also includes an actuator 224 arranged to overcome the spring 222 and bias the blocks 228 together laterally upon actuation. When biased together by the actuator 224, the blocks 228 disengage from the side wall 32 of the reservoir 30 to allow longitudinal movement of the second lateral segment 220 within the reservoir 30. The actuator of the second lateral segment 220 is provided as a shape memory element 224 made of a shape memory material. Alternatively, the actuator 224 can be provided in the form of a solenoid, a piezoelectric element, or another type of actuator capable of bringing the blocks 228 together against the force of the spring 222 upon being actuated. The shape memory element 224 of the second lateral segment 220 comprises a one-way shape memory alloy. However, the shape memory element 224 can be provided as a two-way shape memory alloy. As shown Figs. 2 and 3a-3f, the elongated shape memory element 224 is secured at opposing ends between the two blocks 228 and anchored to the second lateral segment 220 at a midpoint between the two blocks 228. As shown in Fig. 2, the fluid delivery device 10 includes wires 226 connecting the opposite ends of the shape memory element 224 to the processor 50 such that the processor can apply an electrical charge to the element 224.

When a charge is applied to the elongated shape memory element 224, the length of the shape memory element 224 decreases from an uncharged length to a charged length. The shape memory element 224 of the second lateral segment 220 is arranged such that the changeable length of the shape memory element 224 decreasing from an uncharged length to a charged length causes the two blocks 228 to be drawn together against the force of the spring 222, as shown in Figs. 3e and 3f. The plunger assembly is adapted such that the fluid-tight seal between the second lateral segment 220 and the side wall 32 of the reservoir 30 is maintained. When the charge is removed from the elongated shape memory element 224, the spring 222 is allowed to bias the two blocks 228 apart and increase the length of the shape memory element 224 from the charged length to the uncharged length, as shown in Figs. 3a-3d.

Figs. 2 and 3 show the plunger assembly 40 when no charges are applied to the actuators 204, 224, 244, The processor 50 is programmed to provide a predetermined cycle of charges to the actuators 204, 224, 244 of the plunger assembly 40 in order to cause longitudinal advancement of the plunger assembly 40 towards the outlet 36 of the reservoir 30.

For example, during operation of the plunger assembly 40, the actuator 204 of the first lateral segment 200 is charged to pull the blocks 208 of the first lateral segment 200 laterally inwardly away from the side wall 32 of the reservoir 30, as shown in Figs. 3b and 3c, to allow longitudinal movement of the first lateral segment 200. Then the actuator 244 of the longitudinal segment 240 is charged to pull the first lateral segment 200 longitudinally within the reservoir 30 from an initial longitudinal position x1, as illustrated in Figs. 3a-3f, towards the second lateral segment 220 until the first lateral segment 200 is stopped by the longitudinally extending projection 248 of the longitudinal segment 240 at a second longitudinal position x1', as shown in Figs. 3c-3e.

The charge can then be removed from the actuator 204 of the first lateral segment 200 such that the spring 202 of the first lateral segment 200 is allowed to bias the blocks 208 against the side wall 32 and prevent further longitudinal movement of the first lateral segment 200 within the reservoir 30, as shown in Fig. 3d.

Then, the actuator 224 of the second lateral segment 220 is charged to pull the blocks 228 of the second lateral segment 220 laterally inwardly away from the side wall 32 of the reservoir 30, as shown in Figs. 3e and 3f, to allow longitudinal movement of the second lateral segment 220. The plunger assembly is adapted such that the fluid-tight seal between the second lateral segment 220 and the side wall 32 of the reservoir 30 is maintained during this longitudinal movement. The charge is then removed from the actuator 244 of the longitudinal segment 240 to allow the spring 242 of the longitudinal segment 240 to push the second lateral segment 220 longitudinally within the reservoir 30 from an initial longitudinal position x2, as illustrated in Figs. 3a-3f, away from the first lateral segment 200 to a second longitudinal position x2', as shown in Fig. 3f. This longitudinal movement of the second lateral segment 220 causes fluid to be dispensed from the reservoir. The charge can then be removed from the actuator 224 of the second lateral segment 220 such that the spring 222 of the second lateral segment 220 is allowed to bias the blocks 228 against the side wall 32 and prevent further longitudinal movement of the second lateral segment 220 within the reservoir 30, as shown in Fig. 3a.

The longitudinal difference between x2' and x2 is substantially equal to the longitudinal difference between x1' and x1, and substantially equal to the longitudinal difference between the length of the fully elongated and uncharged actuator 244 of the longitudinal segment 240 and the length of the longitudinally extending projection 248 of the longitudinal segment 240. Since both the length of the fully elongated and uncharged actuator 244 and the length of the longitudinally extending projection 248 of the longitudinal segment 240 are predetermined, the longitudinal difference between x2' and x2 is also predetermined.

The cycle of charges applied to the actuators 204, 224, 244 of the plunger assembly 40 as illustrated in Figs. 3b through 3f are successively repeated (through electrical charges provided by the local processor 50) to intermittently advance the plunger assembly 40 longitudinally within the reservoir 30 and produce pulse volumes of fluid flow from the reservoir 30. Thus, one cycle of charges is illustrated in Figs. 3b-3f, and produces a longitudinal displacement of fluid between the plunger assembly and the end wall of the reservoir equal to the longitudinal difference between x2' and x2.

Although not shown, the processor 50 can include capacitors for storing a charge received from the power source 80 for use in providing electrical charges to the actuators 204, 224, 244 of the plunger assembly 40. The fluid delivery device 10 can be calibrated so that a single cycle of charges from the processor 50 causes the dispensing of a predetermine volume of fluid, called a pulse volume (PV), from the reservoir 30. In general, the PV is substantially equal to the longitudinal difference between x2' and x2 multiplied by the cross-sectional area of the reservoir 30.

In this manner, a desired volume to be delivered by the fluid delivery device 10 is dispensed by the application of multiple cycles of charges over a predetermined period. PV's delivered by infusion devices are typically chosen to be small relative to what would be considered a clinically significant volume. For insulin applications at a concentration of one hundred units per microliter (100 units/ml), a PV of less than two microliters, and typically a half of a microliter, is appropriate. If the fluid delivery device 10 is programmed via the remote control device 100 to deliver two units an hour, the processor 50 will deliver forty cycles of charges an hour, or a cycle of charges every ninety seconds, to the actuators 204, 224, 244. Other drugs or concentrations may permit a much larger PV. Various flow rates are achieved by adjusting the time between the cycles of charges. To give a fixed volume or bolus, multiple cycles of charges are given in rapid succession until the bolus volume is reached.

Referring back to Fig. 2, the fluid delivery device 10 can be provided with the fill port 90 connected to the reservoir 30. In the embodiment shown, the fill port 90 includes the needle-pierceable septum 92. Although not shown, the device 10 can further include a sensor, such as a pressure switch, connected to the local processor 50 and adapted and arranged to provide a signal upon the presence of a needle in the fill port 90. The local processor 50, in-turn, can be programmed to apply a charge to the actuators 204, 224 of the first and the second lateral segments 200, 220 whenever it receives a signal from the fill port 90 sensor. Thus when a needle is positioned in the fill port 90, the plunger assembly 40 is disengaged from the side wall 32 of the reservoir 30 to allow the plunger assembly 40 to be moved longitudinally away from the inlet 36 upon fluid being added to the reservoir 30 through a needle inserted into the fill port 90. Alternatively, the device 400 can be provided with a manual actuator, such as a button for a user to push, for applying a charge to the actuators 204, 224 of the first and the second lateral segments 200, 220 during a filling process.

The plunger assembly 40 further includes a case 260 of resiliently flexible material enclosing the longitudinal segment 240 and the first and the second lateral segments 200, 220 in a fluid-tight manner. The case 260 includes a first portion 262 covering the first lateral segment 200, a second portion 264 covering the second lateral segment 220, and a collapsible bellows 266 covering the longitudinal segment 240 and connecting the first and the second portions 262, 264. The case 260 provides a fluid-tight seal between the outermost periphery of the second lateral segment 220 and the side wall 32 of the reservoir 30, such that fluid contained in the reservoir 30 cannot escape between the side wall 32 and the piston assembly 40 and can only exit the reservoir 30 from the outlet 36.

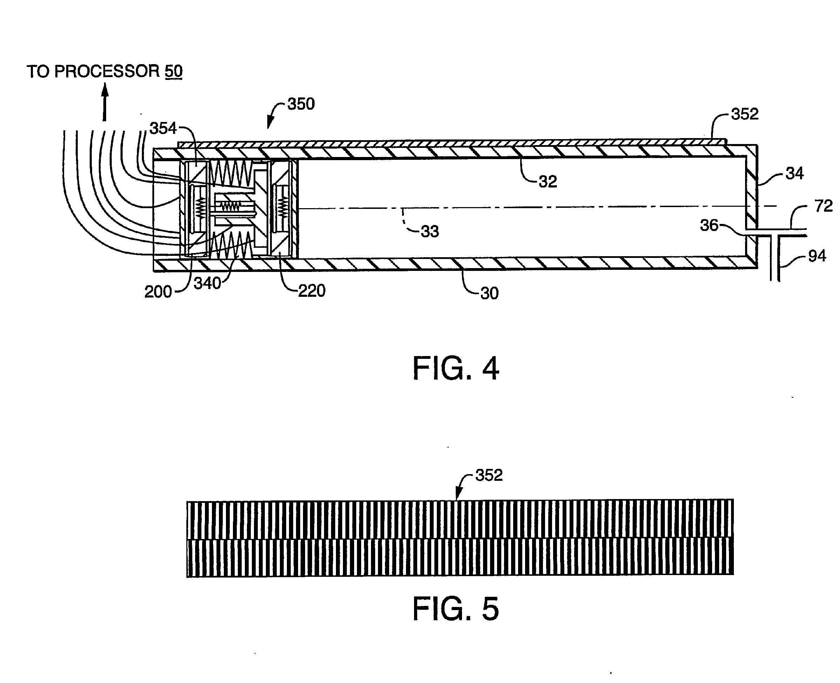

Another exemplary embodiment of a plunger assembly 340 constructed in accordance with the present invention is shown in Fig. 4. Elements of the plunger assembly 340 are similar to elements of the plunger assembly 40 of Figs. 2-3f such that similar elements have the same reference numeral. The plunger assembly 340 and the reservoir 30 of Fig. 4, however, further include a longitudinal position sensor assembly 350.

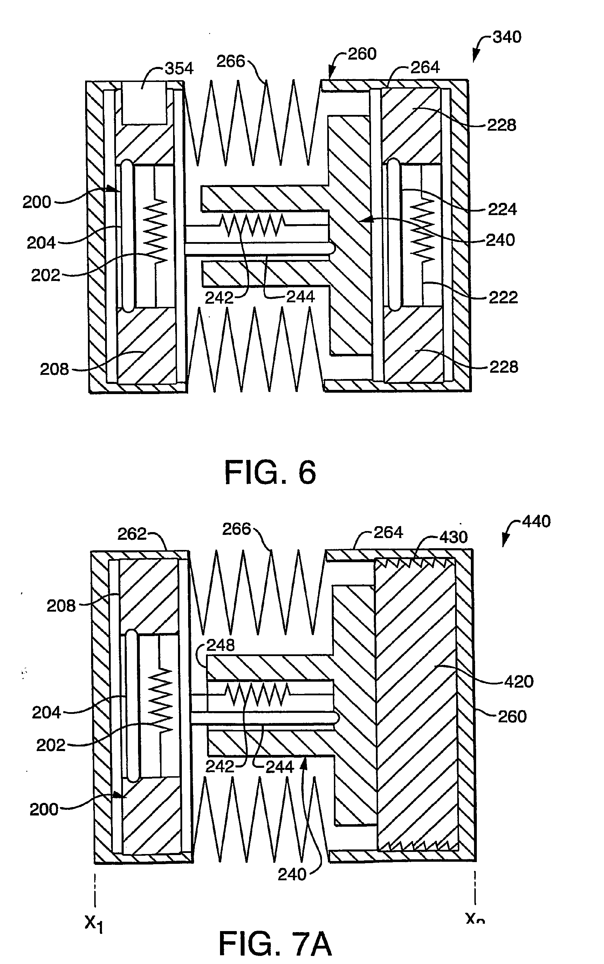

In particular, the longitudinal position sensor assembly 350 includes a barcode 352 secured to the side wall 32 of the reservoir 30 and an optical emitter/receiver 354 mounted in the plunger assembly 340 in alignment with the barcode 352. As shown in Fig. 5, the barcode 352 includes equally spaced alternating bars of reflective and non-reflective material. In the embodiment of the plunger assembly 340 shown in Figs. 4 and 6, the optical emitter/receiver 354 is mounted in the first lateral segment 200. The optical emitter/receiver 354 is connected to the processor 50 of the fluid delivery device and provides a signal whenever the optical emitter/receiver 354 is aligned with one of the reflective bars of the barcode 352. The processor 50 in turn is programmed to determine the relative longitudinal position of the plunger assembly 340 within the reservoir 30 based in part upon the number of signals received from the emitter/receiver 354 in the moving plunger assembly. Thus, the amount of fluid contained in the reservoir 30 can be determined by the processor 50 based on the relative position of the plunger assembly 340 within the reservoir. The processor 50 can also be programmed to determine the amount of fluid dispensed from the reservoir 30 (e.g., microliters) based upon the changing relative position of the plunger assembly 340 within the reservoir 30, and the rate of fluid dispensing (e.g., microliters per hour) based upon the change in the relative position of the plunger assembly 340 within the reservoir 30 and the period of time for that change.

It should be noted that a single row of dark and light bars can be used with single optical receiver/transmitter to detect magnitude of motion. The smaller the bars, the more resolution the motion detector provides. If two rows of offset dark and light bars are used, with a second optical receiver/transmitter, both the magnitude and the direction of motion can be detected (this direction feature can be important if the plunger assembly, for example, sticks and is on the cusp of a dark and light transition and causing a "chattering" back and force signal to the processor and thus false information of infusion).

An additional exemplary embodiment of a plunger assembly 440 constructed in accordance with the present invention is shown in Figs. 7a-7e. Elements of the plunger assembly 440 are similar to elements of the plunger assembly 40 of Figs. 2-3f such that similar elements have the same reference numeral. The plunger assembly 440 of Figs. 7a-7e, however, includes a second lateral segment 420 that does not includes laterally movable blocks, a spring or an actuator. Instead the second lateral segment 420 is simply sized and shaped to frictionally engage the side wall of the reservoir. Moreover, in the embodiment shown the second lateral segment 420 includes outer circumferential ridges 430 shaped and oriented to engage the side wall of the reservoir and substantially prevent movement of the second lateral segment away from the outlet of the reservoir.

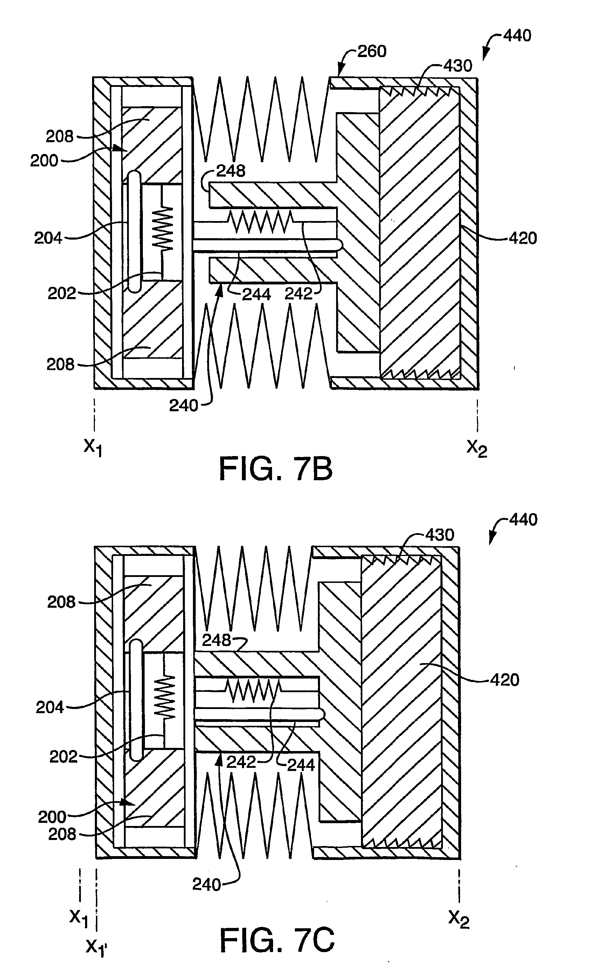

During operation of the plunger assembly 40, the actuator 204 of the first lateral segment 200 is charged to pull the blocks 208 of the first lateral segment 200 laterally inwardly away from the side wall 32 of the reservoir 30, as shown in Figs. 7b and 7c, to allow longitudinal movement of the first lateral segment 200. Then the actuator 244 of the longitudinal segment 240 is charged to pull the first lateral segment 200 longitudinally within the reservoir 30 from an initial longitudinal position x1, as illustrated in Figs. 7a-7e, towards the second lateral segment 420 until the first lateral segment 200 is stopped by the longitudinally extending projection 248 of the longitudinal segment 240 at a second longitudinal position x1' as shown in Figs. 7c-7e.

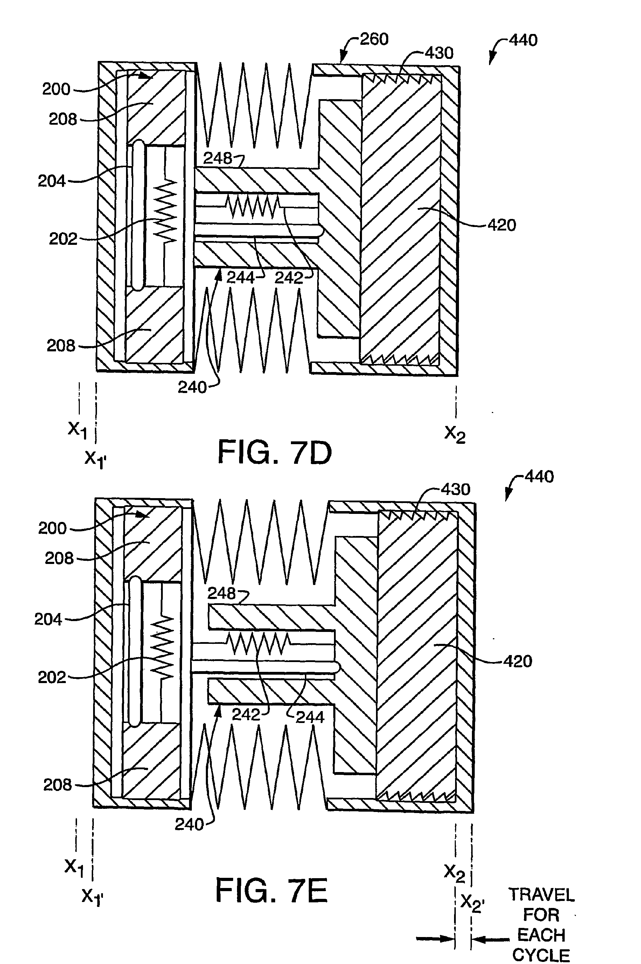

The charge can then be removed from the actuator 204 of the first lateral segment 200 such that the spring 202 of the first lateral segment 200 is allowed to bias the blocks 208 against the side wall 32 and prevent further longitudinal movement of the first lateral segment 200 within the reservoir 30, as shown in Fig. 7d.

Then, the charge is removed from the actuator 244 of the longitudinal segment 240 to allow the spring 242 of the longitudinal segment 240 to push the second lateral segment 420 longitudinally within the reservoir 30 from an initial longitudinal position x2, as illustrated in Figs. 7a-7e, away from the first lateral segment 200 to a second longitudinal position x2', as shown in Fig. 7e.

The frictional engagement force of the second lateral segment 420 against the side wall 32 must be carefully designed to be slightly less than the force generated by the spring 242. Basically, the first lateral segment 200, with the blocks 208 withdrawn, should have a minimal frictional engagement force when compared to the frictional engagement force of the second lateral segment 420. When the longitudinal segment 240 is contracted, the first lateral segment 200 preferentially moves due to the lower frictional forces. After the first lateral segment 200 is locked in place with the release of its blocks 208, the second lateral segment 420 moves forward more slowly than the first lateral segment did due to the constant, higher frictional force acting on the second lateral segment 420.

The cycle of charges applied to the actuators 204, 244 of the plunger assembly 440 as illustrated in Figs. 7b through 7e are successively repeated (through electrical charges provided by the local processor 50) to intermittently advance the plunger assembly 440 longitudinally within the reservoir 30 and produce pulse volumes of fluid flow from the reservoir 30. Thus, one cycle of charges is illustrated in Figs. 7b-7e.

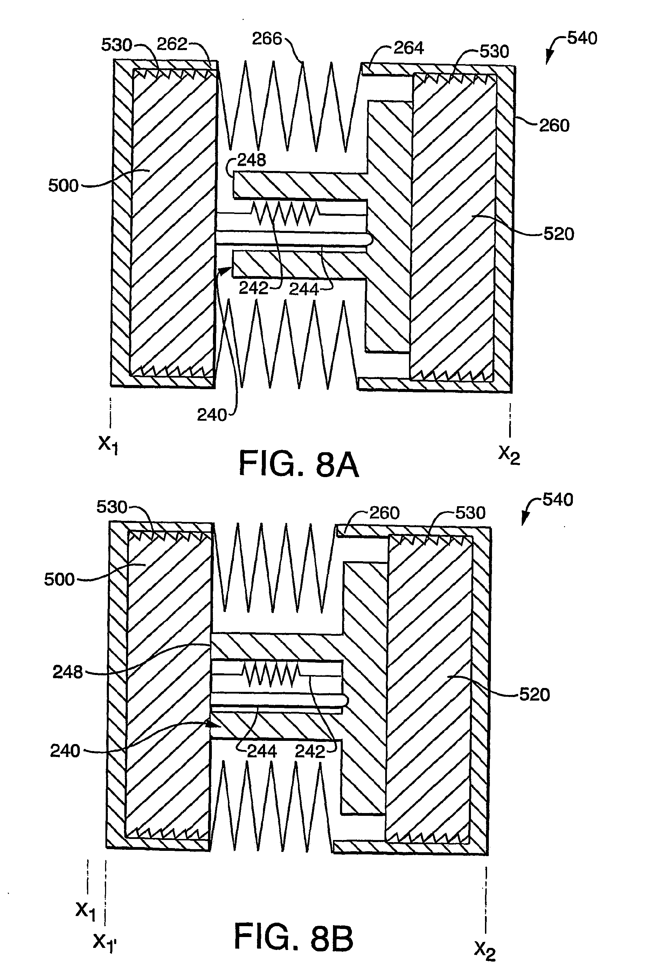

An exemplary plunger assembly 540 the present invention is shown in Figs. 8a-8c. Elements not forming part of of the plunger assembly 540 are similar to elements of the plunger assembly 40 of Figs. 2-3f such that similar elements have the same reference numeral. The plunger assembly 540 of Figs. 8a-8c, however, includes a second lateral segment 520 that does not includes laterally movable blocks, a spring or an actuator, and a first lateral segment 500 that does not includes laterally movable blocks, a spring or an actuator. Instead the first and the second lateral segment 500, 520 are simply sized and shaped to frictionally engage the side wall of the reservoir. Moreover, in the embodiment shown the lateral segment 500, 520 include outer circumferential ridges 530 shaped and oriented to engage the side wall of the reservoir and substantially prevent longitudinal movement of the lateral segments 500, 520 away from the outlet of the reservoir.

During operation of the plunger assembly 540, the actuator 244 of the longitudinal segment 240 is charged to pull the first lateral segment 500 longitudinally within the reservoir 30 from an initial longitudinal position x1, as illustrated in Figs. 8a-8c, towards the second lateral segment 520 until the first lateral segment 500 is stopped by the longitudinally extending projection 248 of the longitudinal segment 240 at a second longitudinal position x1', as shown in Figs. 8b-8c. The actuator 244 of the longitudinal segment 240 is adapted (e.g., sized) to be strong enough to overcome the frictional engagement between the first lateral segment and the side wall of reservoir. Since the circumferential ridges 530 of the second lateral segment 520 prevent longitudinal movement of the second lateral segment 520 away from the outlet of the reservoir, the actuator 244 of the longitudinal segment 240 pulls the first lateral segment 500 towards the second lateral segment 520 without moving the second lateral segment 520.

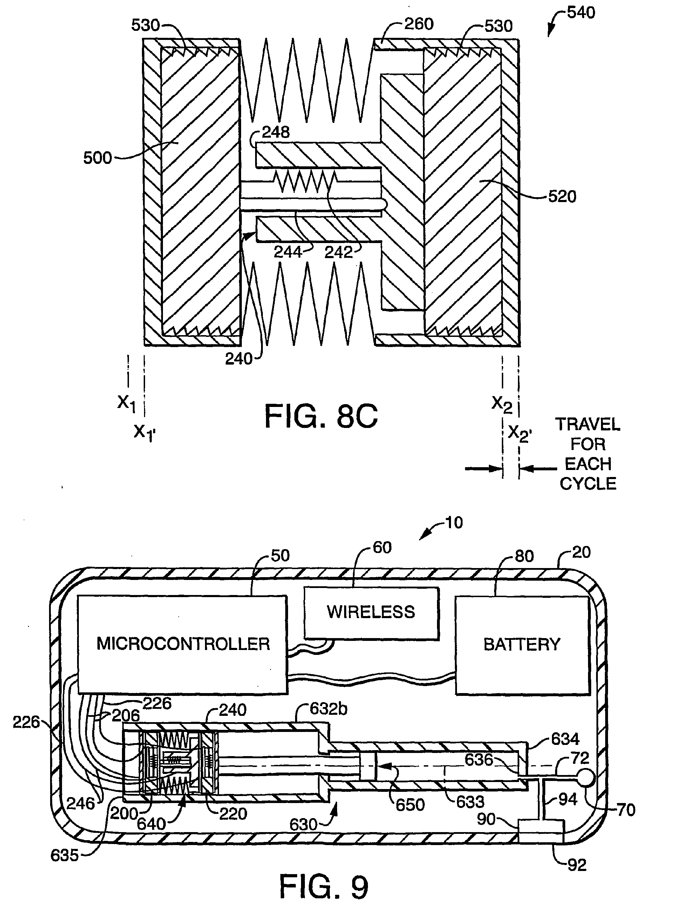

Then, the charge is removed from the actuator 244 of the longitudinal segment 240 to allow the spring 242 of the longitudinal segment 240 to push the second lateral segment 520 longitudinally within the reservoir 30 from an initial longitudinal position x2, as illustrated in Figs. 8a-8c, away from the first lateral segment 500 to a second longitudinal position x2', as shown in Fig. 8c. Since the circumferential ridges 530 of the first lateral segment 500 prevent longitudinal movement of the first lateral segment 500 away from the outlet of the reservoir, the spring 242 of the longitudinal segment 240 pushes the second lateral segment 520 longitudinally away the first lateral segment 500 without moving the first lateral segment 500.

Thus, the single charge applied to the actuator 244 of the plunger assembly 540 as illustrated in Figs. 8a-8c is successively repeated (through electrical charges provided by the local processor 50) to intermittently advance the plunger assembly 540 longitudinally within the reservoir 30 and produce pulse volumes of fluid flow from the reservoir 30. A single charge is illustrated in Figs. 8a-8c.

Fig. 9 shows a fluid delivery device similar to the fluid delivery device of Fig. 2, but including another exemplary embodiment of a reservoir 630 and a plunger assembly 640 constructed in accordance with the present invention for causing fluid to be dispensed from the device. The reservoir 630 and the plunger assembly 640 are similar to the reservoir and the plunger assembly of Fig. 2 such that similar elements have the same reference numerals.

The reservoir 630 is provided with a side wall 632 extending along a longitudinal axis 633 between an open end 635 and an end wall 634 of the reservoir. The end wall 634 includes an outlet, or an opening 636 that functions as an outlet and an inlet. The side wall 632 includes a first section 632a extending from the outlet 636, and a second section 632b extending from the first section 632a to the open end 635 (it should be noted that the reservoirs disclosed herein can be provided with closed ends if desired).

The plunger assembly 640 is received in the second section 632b of the side wall 632 of the reservoir 630. The plunger assembly 640 includes a strut 650 extending along the longitudinal axis 633 of the reservoir 630 and received in the first section 632a of the side wall 632 of the reservoir 630. The strut 650 is shaped and sized such that a fluid-tight seal is generally formed between the strut 650 and the first section 632a of the side wall 632 of the reservoir 630 so that movement of the plunger assembly 640 and the strut 650 towards the end wall 634 of the reservoir 630 forces fluid located between the strut 650 and the end wall 634 through the outlet 636 to the exit port assembly 70.

Features and advantages of the exemplary embodiments of the reservoir 630 and the plunger assembly 640 of Fig. 9 include, but are not limited to, allowing the lateral segments 200, 220 of the plunger assembly 640 to have a cross-sectional dimensions that are different than the cross-sectional dimension of the strut 650, such that a desired pulse volume (PV) produced by the reservoir 630 and the plunger assembly 640 can be further refined. In the exemplary embodiment of Fig. 9, the lateral segments 200, 220 of the plunger assembly 640 are provided with cross-sectional dimensions that are larger than the cross-sectional dimension of the strut 650 (i.e., the first section 632a of the side wall 632 of the reservoir 630 has a cross-sectional dimension that is smaller than a cross-sectional dimension of the second section 632b of the side wall 632). However, the lateral segments 200, 220 of the plunger assembly 640 can be provided with cross-sectional dimensions that are smaller than the cross-sectional dimension of the strut 650 (i.e., the first section 632a of the side wall 632 of the reservoir 630 can be provided with a cross-sectional dimension that is larger than a cross-sectional dimension of the second section 632b of the side wall 632) if desired.

As illustrated by the above described exemplary embodiments, the present invention generally provides a device 10 for delivering fluid, such as insulin for example, to a patient. The device 10 includes an exit port assembly 70, and a reservoir 30 including an outlet 36 connected to the exit port assembly 70 and a side wall 32 extending along a longitudinal axis 33 towards the outlet 36. A plunger assembly (e.g., 40, 340, 440, 540) is received in the reservoir 30 and is movable along the longitudinal axis 33 of the reservoir 30 towards the outlet 36 of the reservoir in order to cause fluid to be dispensed from the reservoir to the exit port assembly 70.

In any event, it should be understood that the embodiments described herein are merely exemplary and that a person skilled in the art may make variations and modifications to the embodiments described without departing from the spirit and scope of the present invention. For example, some linear actuators have a limited contraction distances (i.e., small change in length). A shape memory element for example may be only able to contract approximately 5% of its length upon being charged. In applications where this small change in length is insufficient, various geometric design alternatives can be used to create sufficient linear motion based on the small change in length of the shape memory element. The simplest geometric design alternative, for example, may be to use a longer shape memory element connected back and forth multiple times between the two objects to be pulled together. Alternatively, the shape memory element can be attached to a shorter arm of a lever (or other length versus force exchange mechanism), utilizing the large forces generated by the shape memory element to "exchange" force for increased travel. In any event, all such equivalent variations and modifications are intended to be included within the scope of this invention as defined by the appended claims.

## Claims

1. A device for delivering fluid to a patient, comprising;
an exit port assembly (70);
a reservoir (30) including an outlet connected to the exit port assembly, and a side wall (32) extending along a longitudinal axis towards the outlet (36); and
a plunger assembly (40) received in the reservoir and movable along the longitudinal axis (33) of the reservoir towards the outlet of the reservoir, the plunger assembly including,
a first lateral segment (200) extending laterally with respect to the longitudinal axis of the reservoir and contacting the side wall of the reservoir, the first lateral segment (200) including:
two blocks (208) laterally movable with respect to the longitudinal axis (33) of the reservoir (30),
a spring (202) biasing the two blocks apart and laterally outwardly to frictionally engage the side wall (32) of the reservoir and prevent longitudinal movement of the first lateral segment towards the outlet (36) of the reservoir (30), and
an actuator (204) arranged to overcome the spring of the first lateral segment and bias the blocks together laterally upon actuation;
a second lateral segment (220) positioned between the first lateral segment and the outlet of the reservoir, the second lateral segment (220) extending laterally with respect to the longitudinal axis of the reservoir and contacting the side wall of the reservoir, and longitudinally spaced from the first lateral segment (200), and
a longitudinal segment (240) extending substantially parallel with respect to the longitudinal axis of the reservoir and connecting the first and the second lateral segments (200,220), the longitudinal segment (240) including,
a spring (242) biasing the first and the second lateral segments (200, 220) longitudinally apart, and
an actuator (244) arranged to overcome the spring and bias the first and the second lateral segments (200, 220) longitudinally together upon actuation.

2. A device according to claim 1, wherein the actuator (244) of the longitudinal segment (240) of the plunger assembly (40) comprises an elongated shape memory element having a changeable length decreasing from an uncharged length to a charged length when at least one charge is applied to the shape memory element, the shape memory element connected between the first (200) and the second (220) lateral segments.

3. A device according to claim 2, wherein the shape memory element (244) comprises one-way shape memory material.

4. A device according to claim 2, wherein the shape memory element (244) comprises a wire.

5. A device according to claim 2, wherein the shape memory element (244) is made of a nickel and titanium alloy.

6. A device according to claim 1, wherein the actuator (244) of the longitudinal segment of the plunger assembly comprises a piezoelectric element.

7. A device according to claim 1, wherein the actuator of the longitudinal segment (240) of the plunger assembly (40) comprises a solenoid assembly.

8. A device according to claim 1, wherein the longitudinal segment (240) of the plunger assembly (40) further includes a rigid longitudinal projection (248) of a predetermined length.

9. A device according to claim 1, wherein the plunger assembly (40) is prevented from rotating with respect to the side wall (32) of the reservoir (30).

10. A device according to claim 9, wherein the side wall (32) of the reservoir (30) and the first (200) and the second (220) lateral segments have oval cross-sections. '

11. A device according to claim 1, wherein the plunger assembly (40) further includes a case (260) of resiliently flexible material enclosing the longitudinal segment (240) and the first (200) and the second (220) lateral segments in a fluid-tight manner.

12. A device according to claim 11, wherein the case (260) of the plunger assembly (40) includes a first portion (262) covering the first lateral segment (200), a second portion (264) covering the second lateral segment (220), and a collapsible bellows (266) covering the longitudinal segment (240) and connecting the first and the second portions.

13. A device according to claim 1, wherein the first (200) and the second (220) lateral segments are substantially prevented from moving away from the outlet (36) of the reservoir (30).

14. A device according to claim 13, wherein the first (500) and the second (520) lateral segments include outer circumferential ridges (530) shaped and oriented to engage the side wall of the reservoir (30) and substantially prevent movement of the first and the second lateral segments away from the outlet of the reservoir.

15. A device according to claim 1, wherein the actuator (244) of the longitudinal segment of the plunger assembly (40) is actuated when an electrical charge is applied to the actuator, and the device further comprises:
a local processor (50) electrically connected to the actuator of the longitudinal segment of the plunger assembly and programmed to provide electrical charges to the actuator based upon flow instructions;
a wireless receiver (60) connected to the local processor for receiving flow instructions from a separate, remote control device (100) and delivering the flow instructions to the local processor, and
a housing (20) containing the reservoir, the exit port assembly (70), the plunger assembly, the local processor and the wireless receiver, and wherein the housing is free of user input components for providing flow instructions to the local processor.

16. A device according to claim 1, wherein the reservoir (30) contains a therapeutic fluid.

17. A device according to claim 16, wherein the therapeutic fluid is insulin.

18. A device according to claim 1, wherein the exit port assembly (70) includes a transcutaneous patient access tool.

19. A device according to claim 18, wherein the transcutaneous patient access tool comprises a needle.

20. A device according to claim 1, further comprising a fluid fill port (90) connected to the reservoir (30).

21. A device according to claim 20, further comprising a resealable, needle-pierceable septum.

22. A device according to claim 2, further comprising a local processor (50) connected to ends of the shape memory element (244) and programmed to provide charges to the shape memory element based upon flow instructions.

23. A device according to claim 22, further comprising a power supply (80) connected to the local processor (50).

24. A device according to claim 1, wherein the actuator (204) of the first lateral segment (200) of the plunger assembly comprises an elongated shape memory element having a changeable length decreasing from an uncharged length to a charged length when at least one charge is applied to the shape memory element, the shape memory element connected between the two blocks (208) of the first lateral segment.

25. A device according to claim 24, wherein the shape memory element (204) of the first lateral segment (200) comprises one-way shape memory material.

26. A device according to claim 24, wherein the shape memory (204) element of the first lateral segment (200) comprises a wire.

27. A device according to claim 24, wherein the shape memory element (204) of the first lateral segment (200) is made of a nickel and titanium alloy.

28. A device according to claim 1, wherein the actuator (204) of the first lateral segment (200) of the plunger assembly (40) comprises a piezoelectric element.

29. A device according to claim 1, wherein the actuator (204) of the first lateral segment (200) of the plunger assembly (40) comprises a solenoid assembly.

30. A device according to claim 1, wherein the second lateral segment (220) includes:
two blocks (228) laterally movable with respect to the longitudinal axis of the reservoir;
a spring (222) biasing the two blocks of the second lateral segment apart and laterally outwardly to frictionally engage the side wall (32) of the reservoir and prevent longitudinal movement of the second lateral segment towards the outlet (36) of the reservoir (30); and
an actuator (224) arranged to overcome the spring of the second lateral segment and bias the blocks of the second lateral segment together laterally upon actuation.

31. A device according to claim 30, wherein the actuator (224) of the second lateral segment (220) of the plunger assembly (40) comprises an elongated shape memory element having a chargeable length decreasing from an uncharged length to a charged length when at least one charge is applied to the shape memory element of the second lateral segment, the shape memory element of the second lateral segment connected between the two blocks (228) of the second lateral segment.

32. A device according to claim 31, wherein the shape memory element (224) of the second lateral segment (220) comprises one-way shape memory material.

33. A device according to claim 31, wherein the shape memory element (224) of the second lateral segment (220) comprises a wire.

34. A device according to claim 31, wherein the shape memory element (224) of the second lateral segment (220) is made of a nickel and titanium alloy.

35. A device according to claim 30, wherein the actuator (224) of the second lateral segment (220) of the plunger assembly (40) comprises a piezoelectric element.

36. A device according to claim 30, wherein the actuator (224) of the second lateral segment (200) of the plunger assembly (40) comprises a solenoid assembly.

37. A device according to claim 30, wherein the actuators (204, 224, 244) of the plunger assembly (40) are electrically actuable, and the device further comprises:
a local processor (50) electrically connected to the actuators of the plunger assembly and programmed to provide at least one of a pattern of electrical charges to the actuators based upon flow instructions, the pattern of electrical charges comprising,
providing a charge to the actuator (204) of the first lateral segment (200),
providing a charge to the actuator (244) of the longitudinal segment (240),
removing the charge from the actuator of the first lateral segment,
providing a charge to the actuator (224) of the second lateral segment (220),
removing the charge from the actuator of the longitudinal segment, and
removing the charge from the actuator of the second lateral segment.

38. A device according to claim 37, further comprising;
a wireless receiver (60) connected to the local processor (50) for receiving flow instructions from a separate, remote control device (100) and delivering the flow instructions to the local processor; and
a housing (20) containing the reservoir (30), the exit port assembly (70), the plunger assembly (40), the local processor and the wireless receiver, and wherein the housing is free of user input components for providing flow instructions to the local processor.

39. A system including a fluid delivery device according to claim 15 or 38, and further comprising a remote control device (100) separate from the fluid delivery device and including:
a remote processor;
user interface components (120) connected to the remote processor for allowing a user to provide flow instructions to the remote processor; and
a transmitter (130) connected to the remote processor for transmitting the flow instructions to the receiver of the fluid delivery device.

40. A device according to claim 1, further comprising a sensor (350) for determining the longitudinal position of the plunger assembly (40) within the reservoir (30).

41. A device according to claim 40, wherein the reservoir (30) includes a longitudinally extending barcode (352) and the sensor (350) comprises an optical emitter/receiver mounted on the plunger assembly (40) in alignment with the barcode.

42. A device according to claim 1, wherein the spring (242) comprises a helical compression spring.

43. A device according to claim 1, wherein the spring (202) of the first lateral segment (200) comprises helical compression spring.

44. A device according to claim 30, wherein the spring (222) of the second lateral segment (220) comprises a helical compression spring.

45. A device according to claim 1, wherein a fluid-tight seal exists between an outermost periphery of the second lateral segment (220) and the side wall (32) of the reservoir (30).

46. A device according to claim 1, wherein:
the side wall (632) of the reservoir (630) includes a first section (632a) extending from the outlet (636) of the reservoir parallel with the longitudinal axis and a second section (632b) extending from the first section parallel with the longitudinal axis, and wherein the first section of the side wall has an internal cross-sectional dimension that is unequal to an internal cross-sectional dimension of the second section of the side wall; and
the first (200) and the second (220) lateral segments and the longitudinal segment of the plunger assembly (640) are received in the second section of the side wall of the reservoir, and the plunger assembly further includes strut (650) extending from the second lateral segment and slidingly received in the first section of the side wall of the reservoir, wherein the strut is sized and shaped to provided a substantially fluid-tight seal between the first section of the side wall and the strut.

47. A device according to claim 46, wherein the internal cross-sectional dimension of the first section (632a) of the side wall (632) of the reservoir (630) is smaller than the internal cross-sectional dimension of the second section (632b) of the side wall.

## Patentansprüche

1. Vorrichtung zum Zuführen von Fluid an einen Patienten, die umfasst:
eine Austrittsöffnungsanordnung (70);
einen Behälter (30) mit einem Auslass, der mit der Austrittsöffnung verbunden ist, und einer Seitenwand (32), die sich entlang einer Längsachse in Richtung des Auslasses (36) erstreckt; und
eine Kolbenanordnung (40), die in dem Behälter aufgenommen ist und entlang der Längsachse (33) des Behälters in Richtung des Auslasses des Behälters bewegbar ist, wobei die Kolbenanordnung umfasst:
ein erstes seitliches Segment (200), das sich seitlich in Bezug auf die Längsachse des Behälters erstreckt und die Seitenwand des Behälters berührt, wobei das erste seitliche Segment (200) umfasst:
zwei Blöcke (208), die seitlich in Bezug auf die Längsachse (33) des Behälters (30) bewegbar sind,
eine Feder (202), welche die zwei Blöcke auseinander und seitlich nach außen vorspannt, um reibend in die Seitenwand (32) des Behälters einzugreifen und die Längsbewegung des ersten seitlichen Segments in Richtung des Auslasses (36) des Behälters (30) zu verhindern, und
einen Aktuator (204), der angeordnet ist, um die Feder des ersten seitlichen Segments zu überwinden und die Blöcke nach der Betätigung seitlich aufeinander zu vorzuspannen;
ein zweites seitliches Segment (220), das zwischen dem ersten seitlichen Segment und dem Auslass des Behälters positioniert ist, wobei das zweite seitliche Segment (220) sich seitlich in Bezug auf die Längsachse des Behälters erstreckt und die Seitenwand des Behälters berührt und längs von dem ersten seitlichen Segment (200) beabstandet ist, und
ein Längssegment (240), das sich im Wesentlichen parallel in Bezug auf die Längsachse des Behälters erstreckt und die ersten und zweiten seitlichen Segmente (200, 220) verbindet, wobei das Längssegment (240) umfasst:
eine Feder (242), die die ersten und zweiten seitlichen Segmente (200, 220) längs auseinander vorspannt, und
einen Aktuator (244), der angeordnet ist, um die Feder zu überwinden und die ersten und zweiten seitlichen Segmente (200, 220) nach der Betätigung längs aufeinander zu vorzuspannen.

2. Vorrichtung nach Anspruch 1, wobei der Aktuator (244) des Längssegments (240) der Kolbenanordnung (40) ein längliches Formspeicherelement mit einer änderbaren Länge umfasst, die von einer ungeladenen Länge zu einer geladenen Länge abnimmt, wenn wenigstens eine Ladung auf das Formspeicherelement angewendet wird, wobei das Formspeicherelement zwischen den ersten (200) und den zweiten (220) seitlichen Segmenten verbunden ist.

3. Vorrichtung nach Anspruch 2, wobei das Formspeicherelement (244) ein Einweg-Formspeichermaterial umfasst.

4. Vorrichtung nach Anspruch 2, wobei das Formspeicherelement (244) einen Draht umfasst.

5. Vorrichtung nach Anspruch 2, wobei das Formspeicherelement (244) aus einer Nickel- und Titanlegierung gefertigt ist.

6. Vorrichtung nach Anspruch 1, wobei der Aktuator (244) des Längssegments der Kolbenanordnung ein piezoelektrisches Element umfasst.

7. Vorrichtung nach Anspruch 1, wobei der Aktuator des Längssegments (240) der Kolbenanordnung (40) eine Solenoidanordnung umfasst.

8. Vorrichtung nach Anspruch 1, wobei das Längssegment (240) der Kolbenanordnung (40) ferner einen steifen bzw. starren Längsvorsprung (248) mit einer vorgegebenen Länge umfasst.

9. Vorrichtung nach Anspruch 1, wobei die Kolbenanordnung (40) davon abgehalten wird, sich in Bezug auf die Seitenwand (32) des Behälters (30) zu drehen.

10. Vorrichtung nach Anspruch 9, wobei die Seitenwand (32) des Behälters (30) und die ersten (200) und die zweiten (220) seitlichen Segmente ovale Querschnitte haben.

11. Vorrichtung nach Anspruch 1, wobei die Kolbenanordnung (40) ferner ein Gehäuse (260) aus elastisch flexiblem Material umfasst, welches das Längssegment (240) und die ersten (200) und die zweiten (220) seitlichen Segmente in einer fluiddichten Weise umschließt.

12. Vorrichtung nach Anspruch 11, wobei das Gehäuse (260) der Kolbenanordnung (40) einen ersten Abschnitt (262) umfasst, der das erste seitliche Segment (200) bedeckt, einen zweiten Abschnitt (264), der das zweite seitlichen Segment (240) bedeckt, und einen faltbaren Balg (266), der das Längssegment (240) bedeckt und die ersten und zweiten Abschnitte verbindet.

13. Vorrichtung nach Anspruch 1, wobei die ersten (200) und die zweiten (220) seitlichen Segmente im Wesentlichen davon abgehalten werden, sich von dem Auslass (36) des Behälters (30) weg zu bewegen.

14. Vorrichtung nach Anspruch 13, wobei die ersten (500) und die zweiten (520) seitlichen Segmente Außenumfangsgrate bzw. -rippen (530) umfassen, die geformt und ausgerichtet sind, um in die Seitenwand des Behälters (30) einzugreifen und im Wesentlichen die Bewegung der ersten und der zweiten seitlichen Segmente weg von dem Auslass des Behälters zu verhindern.

15. Vorrichtung nach Anspruch 1, wobei der Aktuator (244) des Längssegments der Kolbenanordnung (40) betätigt wird, wenn eine elektrische Ladung an den Aktuator angelegt wird, und die Vorrichtung ferner umfasst:
einen lokalen Prozessor (50), der mit dem Aktuator des Längssegments der Kolbenanordnung elektrisch verbunden ist und programmiert ist, um basierend auf Durchflussanweisungen elektrische Ladungen an den Aktuator bereitzustellen;
einen drahtlosen Empfänger (60), der mit dem lokalen Prozessor verbunden ist, um Durchflussanweisungen von einer getrennten Fernsteuervorrichtung (100) zu empfangen und die Durchflussanweisungen an den lokalen Prozessor zu liefern; und
ein Gehäuse (20), welches den Behälter, die Austrittsöffnungsanordnung (70), die Kolbenanordnung, den lokalen Prozessor und den drahtlosen Empfänger enthält, und wobei das Gehäuse frei von Benutzereingabekomponenten zum Bereitstellen von Durchflussanweisungen an den lokalen Prozessor ist.

16. Vorrichtung nach Anspruch 1, wobei der Behälter (30) ein therapeutisches Fluid enthält.

17. Vorrichtung nach Anspruch 15, wobei das therapeutische Fluid Insulin ist.

18. Vorrichtung nach Anspruch 1, wobei die Austrittsöffnungsanordnung (70) ein transkutanes Patientenzugangswerkzeug umfasst.

19. Vorrichtung nach Anspruch 18, wobei das transkutane Patientenzugangswerkzeug eine Nadel umfasst.

20. Vorrichtung nach Anspruch 1, die ferner eine mit dem Behälter (30) verbundene Fluidfüllöffnung (90) umfasst.

21. Vorrichtung nach Anspruch 20, die ferner eine wiederverschließbare nadeldurchstechbare Trennwand umfasst.

22. Vorrichtung nach Anspruch 2, die ferner einen lokalen Prozessor (50) umfasst, der mit Enden des Formspeicherelements (244) verbunden ist und programmiert ist, um basierend auf Durchflussanweisungen Ladungen an das Formspeicherelement bereitzustellen.

23. Vorrichtung nach Anspruch 22, die ferner eine mit dem lokalen Prozessor (50) verbundene Stromversorgung (80) umfasst.

24. Vorrichtung nach Anspruch 1, wobei der Aktuator (204) des ersten seitlichen Segments (200) der Kolbenanordnung ein längliches Formspeicherelement mit einer änderbaren Länge umfasst, die von einer ungeladenen Länge zu einer geladenen Länge abnimmt, wenn wenigstens eine Ladung auf das Formspeicherelement angewendet wird, wobei das Formspeicherelement zwischen den zwei Blöcken (208) des ersten seitlichen Segments verbunden ist.

25. Vorrichtung nach Anspruch 24, wobei das Formspeicherelement (204) des ersten seitlichen Segments (200) ein Einweg-Speicherformmaterial umfasst.

26. Vorrichtung nach Anspruch 24, wobei das Formspeicherelement (204) des ersten seitlichen Segments (200) einen Draht umfasst.

27. Vorrichtung nach Anspruch 24, wobei das Formspeicherelement (204) des ersten seitlichen Segments (200) aus einer Nickel- und Titanlegierung gefertigt ist.

28. Vorrichtung nach Anspruch 1, wobei der Aktuator (204) des ersten seitlichen Segments der Kolbenanordnung (40) ein piezoelektrisches Element umfasst.

29. Vorrichtung nach Anspruch 1, wobei der Aktuator (204) des ersten seitlichen Segments (200) der Kolbenanordnung (40) eine Solenoidanordnung umfasst.

30. Vorrichtung nach Anspruch 1, wobei das zweite seitliche Segment (220) umfasst:
zwei Blöcke (208), die seitlich in Bezug auf die Längsachse des Behälters bewegbar sind;
eine Feder (222), welche die zwei Blöcke des zweiten seitlichen Segments auseinander und seitlich nach außen vorspannt, um reibend in die Seitenwand (32) des Behälters einzugreifen und die Längsbewegung des zweiten seitlichen Segments in Richtung des Auslasses (36) des Behälters (30) zu verhindern; und
einen Aktuator (224), der angeordnet ist, um die Feder des zweiten seitlichen Segments zu überwinden und die Blöcke des zweiten seitlichen Segments nach der Betätigung seitlich aufeinander zu vorzuspannen.

31. Vorrichtung nach Anspruch 30, wobei der Aktuator (224) des zweiten seitlichen Segments (220) der Kolbenanordnung (40) ein längliches Formspeicherelement mit einer änderbaren Länge hat, die von einer ungeladenen Länge zu einer geladenen Länge zunimmt, wenn wenigstens eine Ladung auf das Formspeicherelement des zweiten seitlichen Segments angewendet wird, wobei das Formspeicherelement des zweiten seitlichen Segments zwischen den zwei Blöcken (228) des zweiten seitlichen Segments verbunden ist.

32. Vorrichtung nach Anspruch 31, wobei das Formspeicherelement (224) des zweiten seitlichen Segments (220) ein Einweg-Formspeichermaterial umfasst.

33. Vorrichtung nach Anspruch 31, wobei das Formspeicherelement (224) des zweiten seitlichen Segments (220) einen Draht umfasst.

34. Vorrichtung nach Anspruch 31, wobei das Formspeicherelement (224) des zweiten seitlichen Segments (220) aus einer Nickel- und Titanlegierung gefertigt ist.

35. Vorrichtung nach Anspruch 30, wobei der Aktuator (244) des zweiten seitlichen Segments (220) der Kolbenanordnung (40) ein piezoelektrisches Element umfasst.

36. Vorrichtung nach Anspruch 30, wobei der Aktuator (224) des zweiten seitlichen Segments (200) der Kolbenanordnung (40) eine Solenoidanordnung umfasst.

37. Vorrichtung nach Anspruch 30, wobei die Aktuatoren (204, 224, 244) der Kolbenanordnung (40) elektrisch betätigbar sind und die Vorrichtung ferner umfasst:
einen lokalen Prozessor (50), der mit den Aktuatoren der Kolbenanordnung elektrisch verbunden ist und programmiert ist, um basierend auf Durchflussanweisungen wenigstens ein Muster von elektrischen Ladungen an die Aktuatoren bereitzustellen, wobei das Muster elektrischer Ladungen umfasst:
Bereitstellen einer Ladung an den Aktuator (204) des ersten seitlichen Segments (200),
Bereitstellen einer Ladung an den Aktuator (244) des Längssegments (240),
Entfernen der Ladung von dem Aktuator des ersten seitliche Segments,
Bereitstellen einer Ladung an den Aktuator (224) des zweiten seitlichen Segments (220),
Entfernen der Ladung von dem Aktuator des Längssegments, und
Entfernen der Ladung von dem Aktuator des zweiten seitlichen Segments.

38. Vorrichtung nach Anspruch 37, die umfasst:
einen drahtlosen Empfänger (60), der mit dem lokalen Prozessor (50) verbunden ist, um Durchflussanweisungen von einer getrennten Fernsteuervorrichtung (100) zu empfangen und die Durchflussanweisungen an den lokalen Prozessor zu liefern; und
ein Gehäuse (20), das den Behälter (30), die Austrittsöffnungsanordnung (70), die Kolbenanordnung (40), den lokalen Prozessor und den drahtlosen Empfänger enthält, und wobei das Gehäuse frei von Benutzereingabekomponenten zum Bereitstellen von Durchflussanweisungen an den lokalen Prozessor ist.

39. System mit einer Fluidzuführungsvorrichtung nach Anspruch 15 oder 38, die ferner eine Fernsteuervorrichtung (100) getrennt von der Fluidzuführungsvorrichtung umfasst, und das umfasst:
einen entfernten Prozessor;
Benutzerschnittstellenkomponenten (120), die mit dem entfernten Prozessor verbunden sind, um einem Benutzer zum erlauben, Durchflussanweisungen an den entfernten Prozessor bereitzustellen; und
einen Sender (130), der mit dem entfernten Prozessor verbunden ist, um die Durchflussanweisungen an den Empfänger der Fluidzuführungsvorrichtung zu senden.

40. Vorrichtung nach Anspruch 1, die ferner einen Sensor (350) zum Bestimmen der Längsposition der Kolbenanordnung (40) innerhalb des Behälters (30) umfasst.

41. Vorrichtung nach Anspruch 40, wobei der Behälter (30) einen längs verlaufenden Barcode (352) umfasst und der Sensor (350) einen optischen Sender/Empfänger umfasst, der auf der Kolbenanordnung (40) in Ausrichtung mit dem Barcode montiert ist.

42. Vorrichtung nach Anspruch 1, wobei die Feder (242) eine Schneckenspannungsfeder umfasst.

43. Vorrichtung nach Anspruch 1, wobei die Feder (202) des ersten seitlichen Segments (200) eine Schneckenspannungsfeder umfasst.

44. Vorrichtung nach Anspruch 30, wobei die Feder (222) des zweiten seitlichen Segments (220) eine Schneckenspannungsfeder umfasst.

45. Vorrichtung nach Anspruch 1, wobei zwischen einem äußersten Umfang des zweiten seitlichen Segments (220) und der Seitenwand (32) des Behälters (30) eine fluiddichte Dichtung vorhanden ist.

46. Vorrichtung nach Anspruch 1, wobei:
die Seitenwand (632) des Behälters (630) einen ersten Abschnitt (632a) umfasst, der sich von dem Auslass (636) des Behälters parallel zu der Längsachse erstreckt, und einen zweiten Abschnitt (632b), der sich von dem ersten Abschnitt parallel zu der Längsachse erstreckt, und wobei der erste Abschnitt der Seitenwand eine Innenquerschnittabmessung hat, die ungleich einer Innenquerschnittabmessung des zweiten Abschnitts der Seitenwand ist; und
die ersten (200) und die zweiten (220) seitlichen Segmente und das Längssegment der Kolbenanordnung (640) in dem zweiten Abschnitt der Seitenwand des Behälters aufgenommen sind und die Kolbenanordnung ferner eine Strebe (650) umfasst, die sich von dem zweiten seitlichen Segment erstreckt und gleitend in dem ersten Abschnitt der Seitenwand des Behälters aufgenommen ist, wobei die Strebe dimensioniert und geformt ist, um eine im Wesentlichen fluiddichte Dichtung zwischen dem ersten Abschnitt der Seitenwand und der Strebe bereitzustellen.

47. Vorrichtung nach Anspruch 46, wobei die Innenquerschittsabmessung des ersten Abschnitts (632a) der Seitenwand (632) des Behälters (630) kleiner als die Innenquerschnittabmessung des zweiten Abschnitts (632b) der Seitenwand ist.

## Revendications

1. Dispositif pour administrer du fluide à un patient, comprenant :
un ensemble orifice de sortie (70) ;
un réservoir (30) comprenant une sortie raccordée à l'ensemble orifice de sortie, et une paroi latérale (32) s'étendant le long d'un axe longitudinal vers la sortie (36) ; et
un ensemble piston (40) reçu dans le réservoir et mobile le long de l'axe longitudinal (33) du réservoir vers la sortie du réservoir, l'ensemble piston comprenant,
un premier segment latéral (200) s'étendant latéralement par rapport à l'axe longitudinal du réservoir et entrant en contact avec la paroi latérale du réservoir, le premier segment latéral (200) comprenant :
deux blocs (208) mobiles latéralement par rapport à l'axe longitudinal (33) du réservoir (30),
un ressort (202) écartant les deux blocs et les forçant latéralement vers l'extérieur pour qu'ils se mettent en prise par frottement avec la paroi latérale (32) du réservoir et empêchent le mouvement longitudinal du premier segment latéral vers la sortie (36) du réservoir (30), et
un actionneur (204) agencé pour contrecarrer la force du ressort du premier segment latéral et ramener ensemble les blocs latéralement lors de son actionnement :
un second segment latéral (220) positionné entre le premier segment latéral et la sortie du réservoir, le second segment latéral (220) s'étendant latéralement par rapport à l'axe longitudinal du réservoir et entrant en contact avec la paroi latérale du réservoir, et espacé longitudinalement par rapport au premier segment latéral (200), et
un segment longitudinal (240) s'étendant de manière sensiblement parallèle par rapport à l'axe longitudinal du réservoir et raccordant les premier et second segments latéraux (200, 220), le segment longitudinal (240) comprenant,
un ressort (242) écartant les premier et second segments latéraux (200, 220) longitudinalement, et
un actionneur (244) agencé pour contrecarrer la force du ressort et ramener ensemble les premier et second segments latéraux (200. 220) longitudinalement lors de son actionnement.

2. Dispositif selon la revendication 1, dans lequel l'actionneur (244) du segment longitudinal (240) de l'ensemble piston (40) comprend un élément à mémoire de forme allongé ayant une longueur variable diminuant d'une longueur non chargée à une longueur chargée lorsqu'au moins une charge est appliquée à l'élément à mémoire de formes l'élément à mémoire de forme étant relié entre les premier (200) et second (220) segments latéraux.

3. Dispositif selon la revendication 2, dans lequel l'élément à mémoire de forme (244) comprend un matériau à mémoire de forme à sens unique.

4. Dispositif selon la revendication 2, dans lequel l'élément à mémoire de forme (244) comprend un fil.

5. Dispositif selon la revendication 2, dans lequel l'élément à mémoire de forme (244) est fabriqué à partir d'un alliage de nickel et de titane.

6. Dispositif selon la revendication 1, dans lequel l'actionneur (244) du segment longitudinal de l'ensemble piston comprend un élément piézoélectrique.

7. Dispositif selon la revendication 1, dans lequel l'actionneur du segment longitudinal (240) de l'ensemble piston (40) comprend un ensemble solénoïde.

8. Dispositif selon la revendication 1, dans lequel le segment longitudinal (240) de l'ensemble piston (40) comprend en outre une protubérance longitudinale rigide (248) d'une longueur prédéterminée.

9. Dispositif selon la revendication 1, dans lequel l'ensemble piston (40) est dans l'impossibilité de tourner par rapport à la paroi latérale (32) du réservoir (30).

10. Dispositif selon la revendication 9, dans lequel la paroi latérale (32) du réservoir (30) et les premier (200) et second (220) segments latéraux ont des sections transversales ovales.

11. Dispositif selon la revendication 1, dans lequel l'ensemble piston (40) comprend en outre un carter (260) en un matériau flexible élastiquement renfermant le segment longitudinal (240) et les premier (200) et second (220) segments latéraux d'une manière étanche au fluide.

12. Dispositif selon la revendication 11, dans lequel le carter (260) de l'ensemble piston (40) comprend une première partie (262) couvrant le premier segment latéral (200), une seconde partie (264) couvrant le second segment latéral (220), et un soufflet escamotable (266) couvrant le segment longitudinal (240) et reliant les première et seconde parties.

13. Dispositif selon la revendication 1, dans lequel les premier (200) et second (220) segments latéraux sont sensiblement dans l'impossibilité de s'éloigner de la sortie (36) du réservoir (30).

14. Dispositif selon la revendication 13, dans lequel les premier (500) et second (520) segments latéraux comprennent des crêtes circonférentielles externe (530) façonnées et orientées de manière à se mettre en prise avec la paroi latérale du réservoir (30) et empêcher sensiblement les premier et second segments latéraux de s'éloigner de la sortie du réservoir.

15. Dispositif selon la revendication 1, dans lequel l'actionneur (244) du segment longitudinal de l'ensemble piston (40) est actionné lorsqu'une charge électrique est appliquée à l'actionneur, et le dispositif comprend en outre :
un processeur local (50) connecté électriquement à l'actionneur du segment longitudinal de l'ensemble piston et programmé pour fournir des charges électriques à l'actionneur en se basant sur des instructions de débit ;
un récepteur sans fil (60) connecté au processeur local pour recevoir des instructions de débit d'un dispositif de commande à distance séparé (100) et fournissant les instructions de débit au processeur local ; et
un logement (20) contenant le réservoir, l'ensemble orifice de sortie (70), l'ensemble piston, le processeur local et le récepteur sans fil et dans lequel le logement est dépourvu de composants d'entrée utilisateur pour fournir des instructions de débit au processeur local.

16. Dispositif selon la revendication 1, dans lequel le réservoir (30) contient un fluide thérapeutique.

17. Dispositif selon la revendication 16, dans lequel le fluide thérapeutique est de l'insuline.

18. Dispositif selon la revendication 1, dans lequel l'ensemble orifice de sortie (70) comprend un outil d'accès transcutané au patient.

19. Dispositif selon la revendication 18, dans lequel l'outil d'accès transcutané au patient comprend une aiguille.

20. Dispositif selon la revendication 1, comprenant en outre un orifice de remplissage de fluide (90) raccordé au réservoir (30).

21. Dispositif selon la revendication 20, comprenant en outre un septum hermétique pouvant être percé par aiguille.

22. Dispositif selon la revendication 2, comprenant en outre un processeur local (50) connecté aux extrémités de l'élément à mémoire de forme (244) et programmé pour fournir des charges à l'élément à mémoire de forme en se basant sur les instructions de débit.

23. Dispositif selon la revendication 22, comprenant en outre une alimentation électrique (80) connectée au processeur local (50).

24. Dispositif selon la revendication 1, dans lequel l'actionneur (204) du premier segment latéral (200) de l'ensemble piston comprend un élément à mémoire de forme allongé ayant une longueur variable diminuant d'une longueur non chargée à une longueur chargée lorsqu'au moins une charge est appliquée à l'élément à mémoire de forme, l'élément à mémoire de forme étant connecté entre les deux blocs (208) du premier segment latéral.

25. Dispositif selon la revendication 24, dans lequel l'élément à mémoire de forme (204) du premier segment latéral (200) comprend un matériau à mémoire de forme à sens unique.

26. Dispositif selon la revendication 24, dans lequel l'élément à mémoire de forme (204) du premier segment latéral (200) comprend un fil.

27. Dispositif selon la revendication 24, dans lequel l'élément à mémoire de forme (204) du premier segment latéral (200) est fabriqué à partir d'un alliage de nickel et de titane.

28. Dispositif selon la revendication 1, dans lequel l'actionneur (204) du premier segment latéral (200) de l'ensemble piston (40) comprend un élément piézoélectrique.

29. Dispositif selon la revendication 1, dans lequel l'actionneur (204) du premier segment latérale (200) de l'ensemble piston (40) comprend un ensemble solénoïde.

30. Dispositif selon la revendication 1, dans lequel le second segment latéral (220) comprend :
deux blocs (228) mobiles latéralement par rapport à l'axe longitudinal du réservoir;
un ressort (222) éloignant les deux blocs du second segment latéral et les forçant latéralement vers l'extérieur pour qu'ils se mettent en prise par frottement avec la paroi latérale (32) du réservoir et empêchent le mouvement longitudinal du second segment latéral vers la sortie (36) du réservoir (30) ; et
un actionneur (224) agencé pour contrecarrer la force du ressort du second segment latéral et ramener ensemble les blocs du second segment latéral latéralement lors de son actionnement.

31. Dispositif selon la revendication 30, dans lequel l'actionneur (224) du second segment latéral (220) de l'ensemble piston (40) comprend un élément à mémoire de forme allongé ayant une longueur variable diminuant d'une longueur non chargée à une longueur chargée lorsqu'au moins une charge est appliquée à l'élément à mémoire de forme du second segment latéral, l'élément à mémoire de forme du second segment latéral étant relié entre les deux blocs (228) du second segment latéral.

32. Dispositif selon la revendication 31, dans lequel l'élément à mémoire de forme (224) du second segment latéral (220) comprend un matériau à mémoire de forme à sens unique.

33. Dispositif selon la revendication 31, dans lequel l'élément à mémoire de forme (224) du second segment latéral (220) comprend un fil.

34. Dispositif selon la revendication 31, dans lequel l'élément à mémoire de forme (224) du second segment latéral (220) est fabriqué à partir d'un alliage de nickel et de titane.

35. Dispositif selon la revendication 30, dans lequel l'actionneur (224) du second segment latéral (210) de l'ensemble piston (40) comprend un élément piézoélectrique.

36. Dispositif selon la revendication 30, dans lequel l'actionneur (224) du second segment latéral (220) de l'ensemble piston (40) comprend un ensemble solénoïde.

37. Dispositif selon la revendication 30, dans lequel les actionneurs (204, 224, 244) de l'ensemble piston (40) sont actionnables électriquement et le dispositif comprend en outre :
un processeur local (50) connecté électriquement aux actionneurs de l'ensemble piston et programmé pour fournir au moins une d'un modèle de charges électriques aux actionneurs en se basant sur les instructions de débit, le modèle de charges électriques consistant à,
fournir une charge à l'actionneur (204) du premier segment latéral (200),
fournir une charge à l'actionneur (244) du segment longitudinal (240),
retirer la charge de l'actionneur du premier segment latéral,
fournir une charge à l'actionneur (224) du second segment latéral (220),
retirer la charge de l'actionneur du segment longitudinal, et
retirer la charge de l'actionneur du second segment latéral.

38. Dispositif selon la revendication 37, comprenant en outre :
un récepteur sans fil (60) connecté au processeur local (50) pour recevoir des instructions de débit d'un dispositif de commande à distance séparé (100) et communiquer les instructions de débit au processeur vocal et
un logement (20) contenant le réservoir (30), l'ensemble orifice de sortie (70), l'ensemble piston (40), le processeur local et le récepteur sans fil et dans lequel le logement est dépourvu de composants d'entrée utilisateur pour fournir des instructions de débit au processeur local.

39. Système comprenant un dispositif d'administration de fluide selon la revendication 15 ou 38, et comprenant en outre un dispositif de commande à distance (100) séparé du dispositif d'administration de fluide et comprenant :
un processeur à distance;
des composants d'interface utilisateur (120) connectés au processeur à distance pour permettre à un utilisateur de fournir des instructions de débit au processeur à distance ; et
un émetteur (130) connecté au processeur à distance pour transmettre les instructions de débit au récepteur du dispositif d'administration de fluide.

40. Dispositif selon la revendication 1, comprenant en outre un capteur (350) pour déterminer la position longitudinale de l'ensemble piston (40) à l'intérieur du réservoir (3()).

41. Dispositif selon la revendication 40, dans lequel le réservoir (30) comprend un code à barres s'étendant longitudinalement (352) et le capteur (350) comprend un émetteur/récepteur optique monté sur l'ensemble piston (40) en alignement avec le code à barres.

42. Dispositif selon la revendication 1, dans lequel le ressort (242) comprend un ressort de compression hélicoïdal.

43. Dispositif selon la revendication 1. dans lequel le ressort (202) du premier segment latéral (200) comprend un ressort de compression hélicoïdal.

44. Dispositif selon la revendication 30, dans lequel le ressort (222) du second segment latéral (220) comprend un ressort de compression hélicoïdal.

45. Dispositif selon la revendication 1. dans lequel un joint étanche au fluide est présent entre une périphérie la plus externe du second segment latéral (220) et la paroi latérale (32) du réservoir (30).

46. Dispositif selon la revendication 1, dans lequel ;
la paroi latérale (632) du réservoir (630) comprend une première section (632a) s'étendant à partir de la sortie (636) du réservoir parallèle à l'axe longitudinal et une seconde section (632b) s'étendant à partir de la première section parallèle à l'axe longitudinal, et dans lequel la première section de la paroi latérale a une dimension en coupe interne qui est inégale par rapport à une dimension en coupe interne de la seconde section de la paroi latérale ; et
les premier (200) et second (220) segments latéraux et le segment longitudinal de l'ensemble piston (640) sont reçus dans la seconde section de la paroi latérale du réservoir, et l'ensemble piston comprend en outre une entretoise (650) s'étendant à partir du second segment latéral et reçue de manière coulissante dans la première section de la paroi latérale du réservoir, l'entretoise étant dimensionnée et façonnée de manière à fournir un joint sensiblement étanche au fluide entre la première section de la paroi latérale et l'entretoise.

47. Dispositif selon la revendication 46, dans lequel la dimension en coupe interne de la première section (632a) de la paroi latérale (632) du réservoir (630) est inférieure à la dimension en coupe interne de la seconde section (632b) de la paroi latérale.
